# EUROPÄISCHE PATENTSCHRIFT

(11) **EP 0 693 129 B1**
(45) Veröffentlichungstag und Bekanntmachung des Hinweises auf die Patenterteilung: **28.06.2006**
(21) Anmeldenummer: 94913099.1
(22) Anmeldetag: 31.03.1994
(51) Int. Cl.: C12N 15/85, C12Q 1/68, C07K 5/08, A61K 38/55, A61K 31/22, A61K 31/40, A61K 31/135, C07C 271/22, C07C 311/17, C07D 207/06

(54) **VERFAHREN ZUR INHIBIERUNG DER TRANSKRIPTION VON GENEN**
METHOD OF INHIBITING THE TRANSCRIPTION OF GENES
PROCEDE D'INHIBITION DE LA TRANSCRIPTION DE GENES

(30) Priorität: 07.04.1993 DE 4311835
(43) Veröffentlichungstag der Anmeldung: 24.01.1996
(73) Patentinhaber: Boehringer Ingelheim International GmbH, 55218 Ingelheim am Rhein (DE)
(72) Erfinder: BAEUERLE, Patrick, D-79361 Sasbach-Jechtingen (DE); HENKEL, Thomas, San Francisco, CA 94118 (US)
(86) Internationale Anmeldenummer: PCT/EP1994/001014
(87) Internationale Veröffentlichungsnummer: WO 1994/023045

(56) Entgegenhaltungen:
- EP-A- 0 393 457
- WO-A-89/08147
- WO-A-91/01379
- WO-A-92/14696
- WO-A-92/20795
- J. EXP. MEDICINE Bd. 175 , Mai 1992 , ROCKEFELLER UNIV. PRESS, NY,US; Seiten 1181 - 1194 R. SCHRECK ET AL. 'Dithiocarbamates as potent inhibitors of nuclear factor kappaB activation in intact cells' in der Anmeldung erwähnt

## Beschreibung

Die Erfindung bezieht sich auf die Beeinflussung der Transkription von Genen.

Die induzierbare Genexpression beruht u.a. auf der induzierbaren Aktivierung von Proteinen, die die Transkription regulieren, indem sie mit *cis-*regulatorischen DNA-Elementen in Wechselwirkung treten. Die Aktivität dieser als Transkriptionsfaktoren bezeichneten Proteine kann durch ihre de novo-Synthese reguliert werden; diese Strategie erfordert zusätzliche Faktoren, die das Gen für den Transkriptionsfaktor beeinflussen. Im Gegensatz dazu haben posttranslationale Mechanismen der Aktivierung von Transkriptionsfaktoren den Vorteil, schneller ebzulaufen als Mechanismen auf Tranekriptionsebene. Bei der Kontrolle der Aktivität von Transkriptionsfaktoren spielen u.a. inhibitorische Protein-Untereinheiten eine wichtige Rolle. Ein Beispiel dafür ist IP-1, ein Leucinzipper-Protein, das AP-1 inhibiert. In diesem Fall hat die inhibitorische Untereinheit die Funktion eines *trans*-dominanten negativen Regulators auf der Grundlage einer Strukturhomologie mit dem Aktivator. Ein System, in welchem die inhibitorischen Untereinheiten keine Homologie mit den DNA-bindenden Untereinheiten aufweisen, ist das NF-κB-System. Die inhibitorischen Untereinheiten von NF-κB und verwandten Faktoren werden IκB-Proteine genannt, welche die Bindung des Transkriptionsfaktors an DNA reversibel inhibieren (Baeuefle und Baltimore, 1988a,b).

NF-κB, ein heterodimerer Faktor, bestehend aus einer 50 kDa (p50) und einer 65 kDa (p65) DNA-bindenden Untereinheit, ist an der sog. "immediate-early" Aktivierung von Abwehrgenen beteiligt, wenn Zellen primären oder sekundären pathogenen Stimuli ausgesetzt sind (Baeuerle, 1991, Baeuerle und Baltimore, 1991).

Der Transkriptionsfaktor NF-κB (Grimm und Baeuerle, 1993; Blank et al., 1992; Nolan und Baltimore, 1992) wird aktiviert u.a. durch Behandlung von Zellen mit bakteriellen Stimuli (u.a. LPS), Viren (u.a. HIV-Virus Typ I), viralen Produkten, Parasiten, inflammatorischen Zytokinen (u.a. TNF-α, TNF-β, IL-1, IL-2), T-Zellmitogenen (u.a. Lektinen), Proteinsyntheseinhibitoren (u.a. Cycloheximid), physikalischem Streß (UV-Licht, gamma-Strahlung), oxidativem Streß (u.a. Wasserstoffsuperoxid) und Tumorpromotoren (u.a. Phorbolester) (Baeuerle, 1991).

Die Aktivierung von NF-κB als Antwort auf eine große Zahl von pathogenen Stimuli geht durch einen Mechanismus vor sich, der bisher noch nicht zur Gänze aufgeklärt war. An die Aktivierung anschließend erfolgt die Aufnahme von NF-κB in den Zellkern und die Aktivierung der Zielgene durch aktives NF-κB. Es wird angenommen, daß reaktive Sauerstoffverbindungen bei der Aktivierung von NF-κB eine Rolle als Botenstoffe spielen (Schreck et al., 1991).

Es wurde nachgewiesen, daß die Aktivierung von NF-κB mit der Freisetzung der inhibitorischen Untereinheit IkB von einem zytoplasmatischen Komplex mit den DNA-bindenden Untereinheiten p50 und Rel-A (früher als p65 bezeichnet) einhergeht (Baeuerle und Baltimore, 1988a,b). Aufgrund von Experimenten mit Zellextrakten wurde angenommen, daß für die Freisetzung der inhibitorischen Untereinheit IκB-α und die Aktivierung von NF-κB die Phosphorylierung von IκB-α durch Proteinkinase C (PKC) und andere Kinasen verantwortlich sei (Shirakawa und Mizel, 1989; Ghosh und Baltimore, 1990; Kerr et al., 1991).

Folgende Klassen von Genen werden von NF-KB kontrolliert; sie haben ein dekameres DNA-Motiv mit der Konsensussequenz 5'-GGGPuNNTPyCC-3' gemeinsam, das von NF-κB erkannt wird: virale Gene (HIV-1-, Cytomegalie-, SV 40-, Adenovirus), Immunrezeptoren (u.a. leichte Immunglobulin-k-Kette, T-Zellrezeptor B, Adhäsionsmolekül 1), Zytokine (IFN-B, GM-CSF, IL-2, IL-6, TNF-α, TNF-B), Akutphasenproteine (u.a. Angiotensinogen), Transkriptionsfaktoren (u.a."Interferon Regulatory Factor-1", NF-κB-Vorläufer p50), vimentin. (Baeuerle, 1991).

Aufgrund der zahlreichen pathologischen Zustände, an denen eine Aktivierung von Genen durch den Transkriptionsfaktor NF-κB beteiligt ist, besteht Bedarf an Inhibitoren von NF-κB, um die Transkription von Genen, die eine schädliche Wirkung auf den Organismus ausüben, zu inhibieren.

Die bisher zur Inhibierung der Expression von Genen, die der transkriptionellen Kontrolle von NF-κB unterliegen, vorgeschlagenen Lösungen beruhen auf einer Kontrolle der Dissoziation des NF-κB/IκB-α-Komplexes (WO 89/08147 und WO 92/20795).

Der vorliegenden Erfindung lag die Aufgabe zugrunde, den Mechanismus der NF-κB-Aktivierung aufzuklären um davon ausgehend Inhibitoren bereitzustellen zu Können, die spezifisch in diesen Mechanismus eingreifen.

Es wurde überraschend festgestellt, daß IκB-α (früher als MAD-3 bezeichnet; Haskill et al., 1991) nach der Stimulierung von Zellen mit Phorbolester, IL-1, LPS oder TNF-α innerhalb von Minuten verschwindet, ein Vorgang, der zeitlich mit dem Auftreten von aktivem NF-κB zusammenfällt. Die Behandlung von Zellen mit Proteaseinhibitoren oder einem Antioxidans verhindert die induzierbare Abnahme von IκB-α ebenso wie die Aktivierung von NF-κB. Die im Rahmen der vorliegenden Erfindung durchgeführten Versuche haben gezeigt, daß die Aktivierung von NF-κB durch PMA oder andere Stimuli offensichtlich auf einen transient verstärkten Abbau von IκB-α durch eine chymotrypsinähnliche Protease zurückzuführen ist. Weiters wurde gezeigt, daß bestimmte Proteaseinhibitoren die Akkumulierung einer phosphorylierten Form von IκB-α bewirken. Die direkte Phosphorylierung und anschließende Inaktivierung von IκB-α durch PKC oder andere Kinasen sind jedoch, entgegen früheren Annahmen, für eine Inaktivierung von NF-κB in intakten Zellen nicht ausreichend.

Die vorliegende Erfindung bezieht sich auf die Inhibierung der Transkription von Genen in höheren eukaryotischen Zellen durch Hemmung der Aktivierung von NF-κB durch Substanzen, die den proteolytischen Abbau von IκB-α spoztflsch hemmen.

Die Hemmung des proteolytischen Abbaus kann direkt oder indirekt erfolgen. Es ist dabei unerheblich, ob der proteolytische Abbau von IκB-α nach seiner Dissoziation vom p50/p65-Komplex stattfindet oder ob IκB-α durch proteolytischen Abbau als Teil des heterotrimeren Komplexes degradiert wird.

Substanzen, die den proteolytischen Abbau direkt hemmen, sind Proteaseinhibitoren.

Im Rahmen der durchgeführten Versuche wurde eine Wirkung von Serinproteaseinhibitoren auf IκB-α und die NF-κB-Aktivierung festgestellt. Da es sich bei den untersuchten Substanzen um toxische Verbindungen handelt, können diese Verbindungen nicht therapeutisch eingesetzt werden. Für die therapeutische Anwendung kommen Substanzen in Betracht, die eine gezielte Inhibierung des proteolytischen Abbaus von IκB-α bewirken, indem sie möglichst spezifisch die Aktivierung der IκH-α-Protease inhibieren.

Proteaseinhibitoren können auf dem Prinzip ihrer Analogie mit dem Substrat, also IκB-α, funktionieren, indem sie mit IκB-α als Substrat für die Protease konkurrieren.

Ein weiterer Mechanismus, aufgrund dessen ein Inhibitor den proteolytischen Abbau von IκB-α beeinträchtigen kann, wirkt dadurch, daß er die Zugänglichkeit von IκH-α für die Protease zu blockiert, z.B. durch Anlagerung oder durch Herbeiführen einer Konformationsänderung (allosterisch wirkender Inhibitor).

Die im Rahmen der vorliegenden Erfindung erhaltenen experimentellen Ergebnisse weisen darauf hin, daß IκB-α erst dadurch dem proteolytischen Abbau zugänglich wird, daß es posttranslational modifiziert wird, indem es phosphoryliert wird.

Im Rahmen der vorliegenden Erfindung wurden Versuche mit dem Proteaseinhibitor Z-Ile-Glu(OtBu)-Ala-Leucinal (im folgenden auch mit NBIG bezeichnet) durchgeführt, von dem bekannt ist, daß er eine Komponente des Proteasoms hemmt, die chymotrypsinähnliche Spezifität hat. Die Versuche auf den Einfluß dieses Inhibitors auf die Aktivierung von NF-κB sowie die Stabilisierung von IκB-α haben gezeigt, daß dieser Inhibitor die Aktivierung von NF-κB in HeLa-Zellen nach TNF-Stimulierung verhindert, wobei der ID₅₀ in derselben Größenordung lag wie die von diesem Inhibitor früher beobachtete Proteasomhemmung. Dabei weist dieser Inhibitor die Besonderheit auf, daß er eine Akkumulation einer höher phosphorylierten Form von IκB-α bewirkt. Das Auftreten und die Stabilisierung dieser phosphorylierten Form korrelieren zeitlich nicht mit der Aktivierung von NF-κB, was die Annahme bestärkt, daß eine Phosphorylierung in intakten Zellen nicht für die Aktivierung von NF-κB ausreicht. Dies weist darauf hin, daß die induzierbare phosphorylierte Form von IκB-α immer noch im Komplex mit NF-κB vorhanden ist; die Phosphorylierung von IκB-α führt also nicht zu einer Ablösung von IκB-α aus dem Komplex, wie dies zunächst aufgrund von in vitro Experimenten angenommen worden war. Die mit NBIG erhaltenen Ergebnisse deuten insgesamt darauf hin, daß das Proteasom für den selektiven Abbau der phosphorylierten Form von IκB-α verantwortlich ist.

Aufgrund der erhaltenen Ergebnisse kann angenommen werden, daß die Funktion der Phosphorylierung lediglich darin besteht, IκB-α für einen anschließenden raschen proteolytischen Abbau zu markieren. Die durch diese Funktion bedingte Labilität der phosphorylierten Form bietet eine Erklärung dafür, daß eine zellfreie Aktivierung von NF-κB bisher nicht beobachtet werden konnte.

Es kommen als Inhibitoren für die Aktivierung von NF-κB somit auch Substanzen in Betracht, die den proteolytischen Abbau von IκB-α indirekt inhibieren, indem sie die Modifikation, durch die IκB-α in die dem proteolytischen Abbau zugängliche Form gebracht wird, also die Phosphorylierung, verhindern, insbesondere Substanzen, die die dafür verantwortliche Kinase inhibieren.

Eine weitere Möglichkeit für einen indirekten Mechanismus der Proteaseinhibierung besteht darin, daß der Inhibitor die Aktivierung der Protease selbst verhindert, z.B. indem ein hypothetischer Proteaseinhibitor an seiner Freisetzung von der IκB-α-Protease gehindert wird.

Die erfindungsgemäß identifizierbaren Inhibitoren unterscheiden sich in ihrer Wirkungsweise von solchen Substanzen, die eine Aktivierung von NF-κB hemmen, indem sie die Dissoziation von IκB-α vom p50/Rel-A-Heterodimeren inhibieren.

Die bisher vorgeschlagenen Methoden zum Auffinden von Substanzen, die die NF-κB-Aktivierung negativ oder positiv beeinflussen, beruhen darauf, chemische Substanzen auf ihre Fähigkeit zu untersuchen, den Komplex zu stabilisieren oder seine Dissoziation zu fördern (WO 92/20795, WO 89/08147).

Andere Screeningverfahren beruhen darauf, Substanzen auf ihre Fähigkeit zur Modulation der Transkription eines speziell interessierenden Gens zu untersuchen, wobei die Modulation der Transkription auf DNA-Bindungsebene erfolgt (WO 91/01379).

Demgegenüber lag der vorliegenden Erfindung die Aufgabe zugrunde, ausgehend von den über den Mechanismus der NF-κB-Aktivierung gewonnenen Erkenntnissen ein Screeningverfahren bereitzustellen, das das Auffinden von Substanzen mit hoher Spezifität für die NF-κB-Aktivierung ermöglicht, indem Substanzen erfaßt werden, die den proteolytischen Abbau von IκB-α inhibieren.
Die Erfindung betrifft somit ein Verfahren zum Identifizieren von Substanzen, die die NF-κB-Aktivierung inhibieren, welches dadurch gekennzeichnet ist, daß man die Testsubstanzen auf ihre Fähigkeit untersucht, den proteolytischen Abbau von IκB-α spezifisch zu inhibieren, indem man ein IκB-α enthaltendes Substrat in Gegenwart einer Testsubstanz mit einer Zubereitung behandelt, die proteolytische Aktivität für IκB-α aufweist, und bestimmt, ob und in welchem Ausmaß die Testsubstanz den proteolytischen Abbau von IκB-α inhibiert.

Vorzugsweise wird im Anschluß bzw. parallel zu obigem Verfahren (im folgenden "Schritt a)")in Gegenwart der Testsubstanz die NF-κB-Aktivierung in höheren eukaryotischen, insbesondere humanen, mit einem auf NF-κB-Aktivierung ansprechenden Reportergenkonstrukt transformierten Zellen induziert und die Expression des Reportergens gemessen (im folgenden "Schritt b)").

In einer bevorzugten Ausführungsform des Verfahrens wird in Schritt a) als Substrat reines IκB-α in einem sog- "zellfreien" Assay eingesetzt.

Dabei kann z.B. so vorgegangen werden, daß eine definierte Menge an, vorzugsweise rekombinantern, IκB-α (Henkel et al., 1992; Zabel et al. 1993) versehen mit einer meßbaren Markierung, an einen festen Träger gebunden wird.

Auf das immobilisierte IκB-α wird in Gegenwart der Testsubstanz eine Zubereitung aufgebracht, die proteolytische Aktivität für IκB-α aufweist. Bei diesem Präparat kann es sich entweder um einen sog. "aktivierten" Zellextrakt oder, sobald diese verfügbar ist, um die mittels rekombinanten Methoden hergestellte IκB-α-Protease handeln. Falls sich die Annahme bestätigt, daß die IκB-α-Protease ein Bestandteil des Proteasoms (Orlowski, 1990; Rivett, 1989) ist, kann dieses in gereinigter Form oder ein Teil davon als proteolytische Aktivität eingesetzt werden.

Ein aktivierter Zellextrakt, der IκB-α abbauende Aktivität aufweist, wird aus Zellen erhalten, die mit einem bekannten NF-κB-Induktor, wie LPS, Phorbolester, TNF etc. behandelt wurden. Die Induktion der NF-κB-Aktivität bewirkt eine Aktivierung des Systems, das für den proteolytischen Abbau von IκB-α verantwortlich ist. Der eingesetzte Zellextrakt besitzt vorzugsweise im nicht-aktivierten Zustand keine IκB-α-abbauende spezifische Aktivität.

Mit dem aktivierten Zellextrakt werden direkte und indirekte Inhibitoren des proteolytischen IκB-α-Abbaus erfaßt, bei Verwendung von gereinigter IκB-α-Protease werden nur die direkt auf die Protease wirkenden Inhibitoren erfaßt.

Als Zubereitung mit proteolytischer Aktivität für IκB-α kann in den zellfreien Assays ferner eine Präparation aus induzierten Zellen eingesetzt werden, vorzugsweise eine Fraktion des aktivierten Zellextrakts, in der IκB-α-proteolytische Aktivität nachgewiesen wurde. Die proteinchemischen Methoden zur Fraktionierung von Zellextrakten sind dem Fachmann geläufig. Die, z.B. nach Ammoniumsulfatfällung, Molekularsieb- und/oder Ionenaustauschchromatographie, isoelektrischer Fokussierung etc., erhaltenen Fraktionen werden in Vorversuchen auf ihre Fähigkeit zum proteolytischen Abbau von IκB-α untersucht und die entsprechende Fraktion für den Assay eingesetzt, wobei die Menge mit dem Testsubstrat IκB-α so koordiniert wird, daß der Abbau meßtechnisch erfaßt werden kann.

In Abwesenheit einer Testsubstanz (Kontrolle) bzw. in Gegenwart einer Testsubstanz, die keine inhibierende Wirkung auf die proteolytische Aktivität hat, wird das immobilisierte IκB-α proteolytisch abgebaut. Dies macht sich dadurch bemerkbar, daß die Markierung von IκB-α (z.B. eine radioaktive oder eine Fluoreszenzmarkierung), ganz oder teilweise (bei vollständigem oder teilweisem Abbau von IκB-α) vom Träger in den Überstand übergeht. Der Anteil der Markierung, der vom Träger in den Überstand übergegangen ist, ist proportional zum proteolytischen Abbau von IκB-α. In Gegenwart einer Testsubstanz, die den proteolytischen Abbau von IκB-α inhibiert, wird das immobilisierte IκB-α nicht angegriffen, die Markierung bleibt auf dem Träger nachweisbar, es geht keine Markierung in den Überstand.

Alternativ kann in Schritt a) eine vorgegebene Menge an markiertem IκB-α statt an einen Träger immobilisiert auch in löslicher Form vorliegen, wobei die übrigen Testparameter identisch sein können wie bei obiger Testanordnung. Beim Aufbringen eines Präparats mit proteolytischer Aktivität wird das in Lösung befindliche IκB-α in kleinere Bruchstücke abgebaut. Die Bestandteile der Lösung werden dann aufgetrennt, z.B. mittels Dialyse oder Gelfiltration, wobei die Porengröße der Membran oder des Harzes so gewählt wird, daß intaktes IκB-α von den proteolytischen Fragmenten abgetrennt wird. Die markierten Fragmente bzw. das nicht abgebaute markierte IκB-α-Substrat befindet sich nun im Eluat, wobei der Anteil der Markierung auf den Fragmenten an der Gesamtmarkierung proportional dem proteolytischen Abbau von IκB-α ist. In Gegenwart einer Testsubstanz, die die proteolytische Aktivität inhibiert, wird das in Lösung befindliche IκB-α nicht abgebaut, es werden daher keine markierten Fragmente nachweisbar sein.

Eine weitere Alternative für den zellfreien Assay in Schritt a) besteht darin, einen Komplex aus IκB-α mit p50 und/oder p65 herzustellen, wobei die Komponenten vorzugsweise, sobald sämtlich verfügbar, rekombinanten Ursprungs sind und IκB-α eine meßbare Markierung aufweist, diesen Komplex in Gegenwart der Testsubstanz mit einem Präparat mit proteolytischer Aktivität zu behandeln. Kommt die proteolytische Aktivität zur Wirkung, was der Fall ist, wenn die Testsubstanz nicht die Fähigkeit hat, diese Aktivität zu inhibieren, wird IκB-α vom Komplex herunterverdaut, die Markierung befindet sich auf den proteolytischen Fragmenten, die nach Filtrieren auch in diesem Fall im Eluat nachweisbar sind. Bei inhibierender Wirkung der Testsubstanz auf die proteolytische Aktivität bleibt IκB-α mit dem Komplex assoziiert, es geht keine Markierung ins Eluat. Prinzipiell kann der proteolytische Abbau von IκB-α auch anhand der Änderung der Eigenfluoreszenz des Komplexes, die durch die Strukturänderung bedingt ist, verfolgt werden.

Die im Rahmen der vorliegenden Erfindung erhaltenen Befunde, daß der Proteaseinhibitor NBIG die Anhäufung einer phosphorylierten Form von IκB-α bewirkt, ermöglichen eine Variante zellfreier Assays, in denen als Substrat für die Protease die phosphorylierte Form von IκB-α eingesetzt wird. Nachdem zunächst mittels Western Blot mit IκB-α-Antikörpern oder mittels ELISA unter Zusatz von Proteasen, z.B. dem gereinigtem Proteasom oder Protease-enthaltenden Zellfraktionen, überprüft worden ist, ob phosphoryliertes IκB-α tatsächlich die für den Abbau durch eine (konstitutive) Protease labilisierte Form des Substrats darstellt, kann dieses Substrat wie folgt erhalten werden:

Geeignete humane Zellen, die NF-κB und IκB-α exprimieren, beispielsweise HeLa-Zellen oder 293-Zellen (ATCC CRL 1573), werden mit einem Proteaseinhibitor behandelt, von dem sich in Vorversuchen gezeigt hat, daß er eine Akkumulierung der phosphorylierten Form von IκB-α bewirkt, beispielsweise mit NBIG in einer Konzentration von 75 µM. Anschließend werden die Zellen mit einem Induktor für die NF-κB-Aktivierung während einer Zeitspanne, die für diese Aktivierung erforderlich ist, stimuliert, beispielsweise durch TNF-α während 15 Minuten, oder mit PMA oder IL-1. Aus zytoplasmatischen Extrakten von derart behandelten Zellen wird der NF-κB-IκB-α-Komplex, der IκB-α in phosphorylierter Form enthält, gegebenenfalls angereichert, z.B. durch einen Glyzeringradienten, durch Gelfiltration oder Ionenaustauschchromatographie, und von überschüssigem NBIG sowie von der durch diesen inaktivierten endogenen Protease abgetrennt. Um IκB-α vollständig in die phosphorylierte Form überzuführen, wird vorzugsweise ein Phosphatasehemmer wie Okada-Säure in einer geeigneten Konzentration, beispielsweise 100 nmol, zugesetzt.

Das so erhaltene phosphorylierte IκB-α, das das Substrat für die Protease darstellt, die zur Aktivierung von NF-κB führt, kann in den oben angeführten Assays eingesetzt werden, um Inhibitoren für die Aktivierung von NF-κB zu finden (alternativ kann IκB-α in phosphorylierter Form bereitgestellt werden, indem rekombinantes IκB-α in vitro phosphoryliert wird). Das Prinzip des Assays bleibt dasselbe: bei Abwesenheit von Proteaseinhibitoren (ohne Zusatz von Testsubstanzen oder in Gegenwart von Testsubstanzen ohne inhibitorische Wirkung) wird das phosphorylierte Substrat abgebaut, in Gegenwart von Inhibitoren bleibt die phosphorylierte Form des NF-κH-IκB-α-Komplexes nachweisbar.

Um in Schritt a) erhaltene positive Ergebnisse hinsichtlich der Spezifität der Testsubstanzen für den proteolytischen Abbau von IκB-α einengen zu können, sind zusätzliche Kontrollen erforderlich:

So wird z.B. ein in Schritt a) aufgefundener Inhibitor daraufhin untersucht, ob er die Aktivität anderer Proteasen direkt beeinträchtigt. Dabei wird zweckmäßig so vorgegangen, daß man die Wirkung des Inhibitors auf die Aktivität einer Protease, die ein von IκB-α verschiedenes Substrat hat, untersucht.

Um zu untersuchen, ob der Inhibitor indirekt, also auf das IκB-α-Substrat, wirkt, wird zweckmäßig der Effekt des Inhibitors mit einer bekannten Protease untersucht, z.B. Chymotrypsin (dazu muß in Vorversuchen sichergestellt worden sein, daß der Inhibitor die Aktivität dieser Kontroll-Protease nicht direkt beeinflußt).

Die im Rahmen der vorliegenden Erfindung erhaltenen Ergebnisse deuten darauf hin, daß die IκB-α-Protease eine Chymotrypsin-ähnliche Serinprotease ist. Nach Bestätigung dieses Befundes im zellfreien System durch den Inhibitor TPCK kann das erfindungsgemäße Screeningsverfahren dadurch erweitert werden, daß schwerpunktmäßig Abkömmlinge von Serinproteaseinhibitoren getestet werden. Als Kontrolle wird zweckmäßig die Wirkung des aufgefundenen Inhibitors einerseits auf die Serinprotease, z.B. Chymotrypsin, andererseits auf die IκB-α-Protease untersucht. (Für den Fall, daß sich dieser Befund wider Erwarten nicht bestätigt, kann prinzipiell analog vorgegangen werden, indem schwerpunktmäßig Derivate von Inhibitoren der Proteaseklasse getestet werden, zu der die IκB-α-Protease definitiv zuzuordnen ist, und die entsprechenden Kontrollen durchgeführt werden.)

Die Wahl der am besten geeigneten Assay-Variante wird an Hand von Vorversuchen getroffen, z.B. ob IκB-α allein, gegebenenenfalls in phosphorylierter Form, oder in Assoziation mit seinen Komplexpartnern p50 und/oder p65 als Testsubstrat dient, ebenso die spezifischen Assay-Bedingungen.

Die übrigen Assay-Bedingungen, wie Art und Konzentration des Induktors, Dauer und Bedingungen der Induktion, Zellaufschluß, Menge an IκB-α bzw. seiner gegegenenfalls vorhandenen Komplexpartner, Träger sowie Bindung von IκB-α bzw. der Komplexpartner an den Träger (Bindung an Mikrotiterplatten mittels bivalenten Crosslinkern wie Glutardialdehyd, an Sepharose über Bromcyan, etc.), Markierung (gekoppeltes Enzym, Radioisotop, fluoreszierende, chemilumineszierende oder biolumineszierende Substanz, etc.), Dauer der Behandlung, Aufbau und Anzahl der Kontrollen, etc. werden mittels Vorversuchen optimiert.

Die Assaybedingungen in Schritt a) werden vorzugsweise so gewählt, daß der Assay automatisiert werden kann.

Für den Fall, daß im mit Zellextrakt durchgeführten Assay die Spezifität des Inhibitors hinsichtlich der Inhibierung der IκB-α-Protease nicht zweifelsfrei geklärt wurde, wird im Anschluß an den zellfreien Assay bzw. parallel dazu ein sog. zellulärer Assay (Schritt b) durchgeführt, in welchem man die Wirkung der Testsubstanz auf die Aktivierung von NF-κB in der intakten Zelle bestimmt. Vorzugsweise wird Schritt b) in jedem Fall durchgeführt, um eine im zellfreien Assay aufgefundene den proteolytischen Abbau von IκB-α inhibierende Wirkung der Testsubstanz in der intakten Zelle zu bestätigen.

Das Reportergen-Konstrukt, mit dem die in b) eingesetzte Testzelle transformiert ist, wird im folgenden als "Sensor-DNA" bezeichnet. Darunter wird ein DNA-Konstrukt verstanden, das ein Reportergen unter Kontrolle von regulatorischen Sequenzen enthält, die auf Aktivierung von NF-κB ansprechen, indem sie mindestens eine Bindungssequenz für NF-κB enthalten. Bei Aktivierung von NF-KB bindet NF-κB an die Erkennungssequenz, worauf die Expression des Reportergens in Gang gesetzt wird, die ein meßbares Signal liefert.

Die Sensor-DNA befindet sich vorzugsweise auf einem Plasmid, das sich in einem geeigneten Wirtsorganismus, vorzugsweise E. coli, in hoher Kopienzahl vermehren läßt und nach Transfektion in Säugetierzellen und Integration ins Wirtsgenom die Expression eines Reportergens unter Kontrolle von regulatorischen Elementen ermöglicht. Es handelt sich dabei bevorzugt um einen Shuttle-Vektor, der eine Expressionskassette für das Reportergen (Sensor DNA) und einen selektionierbaren Marker für Säugetierzellen sowie mindestens einen Replikationsursprung und einen Marker für die Vermehrung und Selektion in E.coli enthält.

Zur Herstellung von permanenten Zellinien, die die Sensor-DNA stabil in ihr Genom integriert enthalten, enthält der Vektor einen dominanten Selektionsmarker. Die Verwendung eines bestimmten Selektionsmarkers ist nicht kritisch, geeignet sind z.B. das Gen für Neomycin-Phosphotransferase (neo), das eine Resistenz gegenüber dem Antibiotikum Geneticin (G-418) verleiht (Southern und Berg, 1982), das DHFR-Gen (Dihydrofolatreduktase) für DHFR-defiziente Zellen, das Gen für Xanthin-Guanin-Phosphoribosyltransferase (gpt), das Resistenz gegen Mycophenolsäure verleiht (Mulligan und Berg, 1981) oder das Hygromycin-B-Phosphotransferase-Gen (hph; Gritz und Davies, 1983). Beispiele für Promotoren, die das Selektionsmarker-Gen treiben, sind der SV40-Early-Promotor, der Cytomegalovirus-Promotor (CMV-Promotor), der Promotor des Thymidin-Kinase-Gens des Herpes simplex-Virus (TK-Promotor), der Rous Sarcoma Virus (RSV) long terminal repeat (LTR). Die Plasmide werden vorzugsweise derart konstruiert, daß einzelne wichtige Elemente, wie das Reportergen, der Promotor für das Reportergen, die regulatorischen Sequenzen für den Selektionsmarker, einfach ausgetauscht oder verändert werden können, um etwaigen geänderten Anforderungen, die sich aus dem speziellen Anwendungsfall ergeben, z.B. aufgrund der Verwendung einer anderen Zellinie, entsprechen zu können. Derartige Maßnahmen bestehen z.B. darin, vor den/die Promotor(en) oder vor das Reportergen Multiklonierstellen einzubauen, um die Klonierung von regulatorischen, den Promotor modulierenden Sequenzen bzw. von verschiedenen Reportergenen zu ermöglichen.

Bei der Auswahl eines geeigneten Reportergens wird davon ausgegangen, einen, vorzugsweise nichtradioaktiven, automatisierbaren Assay mit hoher Empfindlichkeit bereitzustellen.

Im Rahmen der vorliegenden Erfindung können grundsätzlich sämtliche Reportergene eingesetzt werden, die diese Voraussetzungen erfüllen:

Alkalische Phosphatase kann bei Verwendung eines chemilumineszierenden Substrats mit hoher Empfindlichkeit gemessen werden, weist allerdings den Nachteil auf, daß viele Säugetierzellen dieses Enzym relativ stark exprimieren. Es kommt daher als Reportergen im allgemeinen nur für solche Zellinien in Betracht, die es nicht oder nur schwach exprimieren.

Die Expressionsprodukte des β-Galaktosidase- und des β-Glucuronidase-Gens können das entsprechende Methylumbeliferyl-Galaktosid bzw. -Glucuronid unter Bildung fluoreszierender Gruppen spalten. Diese Enzymreaktionen werden mit Hilfe etablierter Fluoreszenz-Assays verfolgt (Wieland et al., 1985; Kricka, 1988).

Die Expression von Chloramphenicol-Actetyltransferase (CAT) ist zwar relativ empfindlich nachweisbar, der Assay weist jedoch u.a. den Nachteil auf, daß er radioaktiv und schwer automatisierbar ist (Hartmann, 1991).

Bevorzugt wird im Rahmen der vorliegenden Erfindung als Reportergen das für Photinus pyralis-Luciferase kodierende Gen (De Wet et al., 1987) verwendet. Dieses Enzym weist die Vorteile auf, daß es mit seinem Substrat Luciferin unter Zugabe von ATP Bioluminiszenz in hoher Ausbeute erzeugt, die mit Hilfe etablierter, automatisierbarer Methoden gemessen werden kann, und daß dieses Enzym von Säugetierzellen nicht endogen produziert wird. Weiters hat Luciferase eine relativ kurze in vivo Halbwertszeit und ist auch in hohen Konzentrationen nicht toxisch (Hartmann, 1991; Brasier et al., 1989). Die Messung der Aktivität der Firefly-Luciferase mittels Biolumineszenz stellt eine der empfindlichsten Methoden einer Enzymbestimmung dar. Daher und aufgrund des Fehlens von Luciferaseaktivität in normalen Säugetierzellen ist dieses Enzym als Reportergen besonders geeignet (Subramani und DeLuca, 1987).

Alternativ kann als Reportergen das für das Enzym Apoaequorin der Qualle Aequoria victoria kodierende Gen (Tanahashi et al., 1990) verwendet werden. Dieses Enzym weist die Vorteile auf, daß es mit seinem Co-Faktor Coelenterazin nach Bindung von Kalziumionen Bioluminiszenz in hoher Ausbeute erzeugt, die mit Hilfe etablierter, automatisierbarer Methoden gemessen werden kann. Ein weiterer Vorteil ist, daß dieses Enzym von Säugetierzellen nicht endogen exprimiert wird.

Das Reportergen wird von einem Promotor getrieben, der unabhängig von anderen Faktoren ist und die Induktion der Genexpression gut sichtbar werden läßt, z.B. der Thymidinkinase-Promotor. An den Promotor wird die Anforderung gestellt, daß das von ihm getriebene Reportergen korrekt und meßbar transkribiert wird. Es kommen als Promotoren Minimalpromotoren, z.B. der Minimal-TK-Promotor und der Minimal-β-Globin-Promotor in Frage. Für die im Rahmen der vorliegenden Erfindung durchgeführten zellulären Assays wurde der -105 - +52 TK-Promotor verwendet, der relativhohe Basalaktivität aufweist und sich gut stimulieren läßt; in Vorversuchen haben sich auch der -30 und der -87 Minimal-TK-Promotor als geeignet erwiesen.

Als Bindungsmotiv für NF-κB ist grundsätzlich jedes Motiv der Konsensussequenz 5'-GGGPuNNTPyCC-3'geeignet, z.B. kann das gut charakterisierte Motiv der leichten Immunglobulin κ-Kette, das die Sequenz 5'-GGGACTTTCC-3'hat, eingesetzt werden. Das NF-κB-Bindungsmotiv liegt zweckmäßig in mindestens zwei Kopien in geringem Abstand voneinander (ca. 10 Nukleotide) vor; im Rahmen der durchgeführten Versuche wurden sechs κB-Motive verwendet.

Grundsätzlich können NF-κB-Bindungssequenzen aus sämtlichen Genen, die durch NF-κB kontrolliert werden, verwendet werden. (Baeuerle, 1991). Falls die Gene neben der NF-κB-Bindungssequenz noch Bindungsdomänen für andere Transkriptionsfaktoren enthalten, werden diese vorzugsweise entfernt.

Als Kontrollzellen dienen Zellen, die in ihrem Reportergen kein DNA-Bindungsmotiv für NF-κB enthalten. Die Kontrollzellen erlauben es, einen unspezifischen Effekt der Substanz auf die Genexpression aufzufinden; ein etwa in der Kontrollzelle erhaltenes Signal würde von der Basistranskription herrühren und müßte daher von dem in der Testzelle erhaltenen Signal abgezogen werden.

Die Testzellen müssen die Bedingung erfüllen, daß in ihnen endogenes NF-KB gut aktivierbar ist, NF-κB soll in diesen Zellen nicht konstitutiv aktiv sein.

Die Zellen müssen weiters mit Substanzen, die NF-κB aktivieren, z.B. mit PMA, LPS, H₂O₂, UV, induzierbar sein.

Zellen können auf ihre Eignung als Testzellen untersucht werden, indem sie mit der Sensor-DNA transformiert werden und die Kinetik der Induktion der Reportergenexpression die Konzentrationsabhängigkeit des Induktors der Zellen bestimmt wird.

Im Hinblick auf ein automatisiertes Verfahren sollten die Zellen möglichst adhärent sein.

Mit dem erfindungsgemäßen Verfahren werden sowohl direkt als auch indirekt den proteolytischen Abbau von IκB-α und somit die Aktivierung von NF-κB inhibierende Substanzen erfaßt, also auch Substanzen, die die Phosphorylierung von IκB-α inhibieren; es ist für die inhibitorische Wirklung-unerheblich, ob die Protease selbst spezifisch für IκB-α ist, wesentlich ist die Spezifität hinsichtlich der Aktivierung.

Substanzen, die den proteolytischen Abbau von IκB-α spezifisch inhibieren, können zur Behandlung von pathologischen Zuständen, an denen eine Expression von Genen, die durch den Transkriptionsfaktor NF-κB kontrolliert werden, beteiligt ist, eingeseht werden.

Zu pathologischen Zuständen, die durch die schädliche Wirkung der durch NF-κB-Aktivierung hervorgerufenen Genexpression verursacht werden, zählen u.a-entzündliche Erkrankungen, die mit einer Aktivierung von T-Zellen, Makrophagen oder B-Zellen einhergehen, toxischer Schock, Erkrankungen nach Infektion mit Viren, die κB-Motive besitzen, UV-Schäden (Sonnenbrand), Strahlenschäden, Verbrennungen, TransplantatabstoBungen, Reperfusionsschäden.

Für die therapeutische Anwendung werden die nach dem erfinaungsgemäßen Verfahren identifizierten Substanzen, die in weiterer Folge in für die Entwicklung von Arzneimitteln üblicher Weise hinsichtlich ihrer pharmakologischen Eigenschaften näher charakterisiert werden, z.B. in sekundären Screenings und im Tierversuch, je nach Applikationsform mit geeigneten pharmazeutisch annehmbaren Träger- und Hilfsstoffen, die die Bioverfügbarkeit der therapeutisch wirksamen Substanzen gewährleisten und keine schädigende Wirkung auf den Organismus ausüben, formuliert. Methoden zur Formulierung pharmazeutischer Zubereitungen sind einschlägigen Handbüchern entnehmbar, beispielhaft wird auf Remington's Pharmaceutical Sciences, 1980, Bezug genommen.

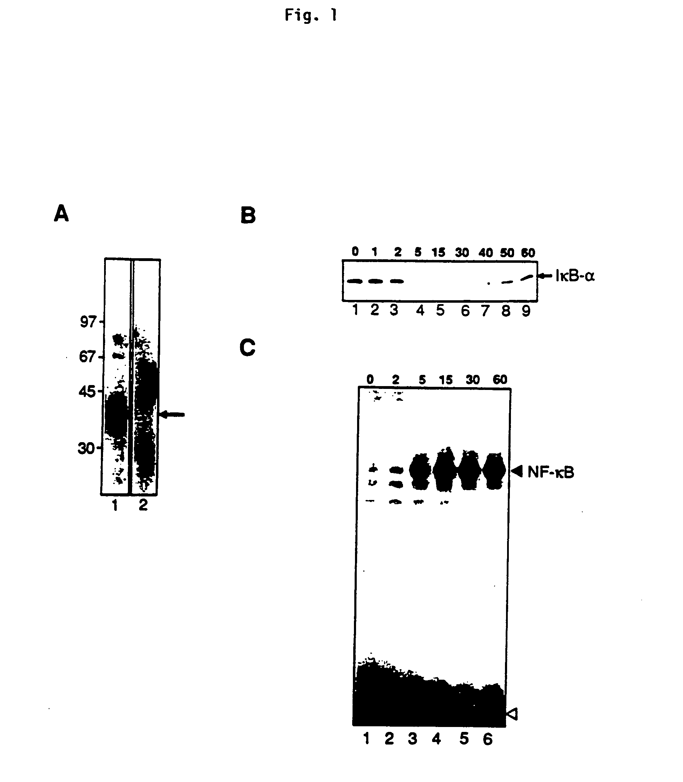

Im Rahmen der vorliegenden Erfindung wurde die Wirkung von Proteaseinhibitoren auf den proteolytischen Abbau von IκB-α festgestellt. Die Inhibierung der NF-κB-Aktivierung in induzierten, mit einem auf NF-κB ansprechenden Luciferasegenkonstrukt transformierten Testzellen wurde bestätigt, ebenso die Inhibierung der NF-κB-regulierten Expression von IL-6 und IL-8. Im Rahmen der vorliegenden Erfindung wurde das Schicksal der NF-κB-spezifischen inhibitorischen Untereinheit IκB-α nach der Behandlung von Zellen mit NF-κB-aktivierenden Stimuli verfolgt. Dazu wurde hochgereinigtes rekombinantes humanes IκB-α verwendet, um ein Kaninchenantiserum zu erhalten; spezifisches IgG wurde durch immobilisiertes IκB-α affinitätsgereinigt. Das IκB-α-spezifische polyklonale IgG erkannte in Western Blots in einem Gesamtzellextrakt von Maus 70Z/3 prä-B Zellen eine einzige 38 K-Bande mit der vorhergesehenen Größe von IκB-α (Fig. 1A, Spur 1). Diese Bande wurde mit einem Kontollantikörper (anti-Kaninchen IgG-Antikörper) nicht erkannt (Fig. 1A, Spur 2). Zwischen 2 und 5 min nach der Zugabe von Phorbol-12-Myristat-13-Acetat (PMA) zu den 70Z/3 Zellkulturen verschwand die IκB-α-Bande beinahe vollständig aus den Zellen (Fig. 1B, vergleiche Spuren 3 und 4). Die Zellen zeigten daraufhin keine IκB-α-Immunreaktivität, bis sie 40 min nach Stimulierung der Zellen wieder auftrat (Fig. 1B, Spur 7). Aliquots derselben Zellextrakte wurden mittels elektrophoretischem Mobility Shift Assay (EMSA) auf KBspezifische DNA-Bindungsaktivität analysiert. Wie aus Fig. 1C ersichtlich ist (vgl. die Spuren 2 und 3), überlappte das Verschwinden von IκB-α exakt mit dem Auftauchen der NF-κB-DNA-Bindungsaktivität, was auf einen kausalen Zusammenhang zwischen diesen beiden Ereignissen schließen läßt.

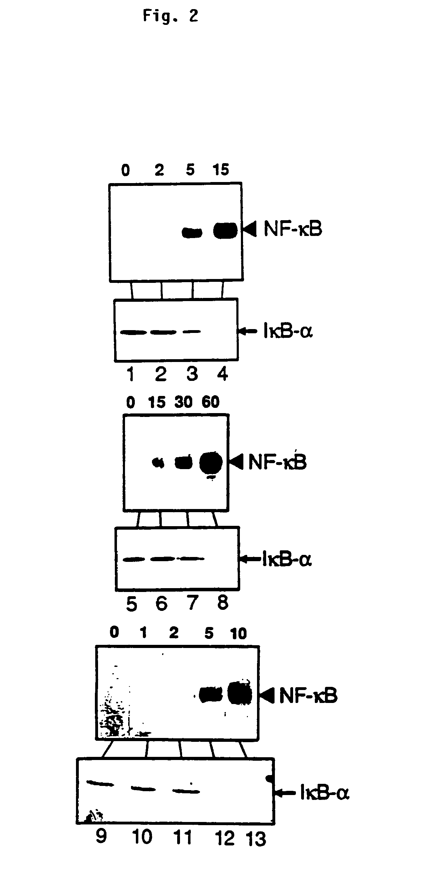

NF-κB kann in 70Z/3 Zellen auch durch Behandlung mit IL-1β und LPS potent aktiviert werden. Die Aktivierung von NF-κB durch TNF-α kann in HeLa-Zellen studiert werden. Wie in Fig. 2 gezeigt wird, induzierten IL-1β, LPS und TNF-α sämtlich einen Zerfall von IκB-α in 70Z/3- bzw. HeLa-Zellen. Es gab jedoch kinetische Unterschiede hinsichtlich des Beginns des Verschwindens von IκB-α. Das meiste IκB-α war bereits 5 min nach der Stimulierung von 70Z/3 Zellen mit PMA (Fig. 1B) bzw. von HeLa-Zellen mit TNF-α (Fig. 2) zerfallen. Der Zerfall begann bei Stimulierung von 70Z/3 Zellen mit IL-1β etwas später; es war nach 5 min Stimulierung mehr IκB-α übrig als bei den mit PMA oder TNF-α behandelten Zellen (Fig. 2B) beobachtet worden war. In LPSstimulierten 70Z/3 Zellen wurde das meiste IκB-α nicht vor 30 min ab Induktion abgebaut (Fig. 2). Wiederum, und zwar bei allen drei Induktoren, ging das Verschwinden von IκB-α unmittelbar mit dem Auftauchen der NF-κB-DNA-Bindungsaktivität in den Zellen einher. Diese Beobachtungen zeigen, daß vier verschiedene Induktoren von NF-κB einen gemeinsamen Mechanismus der NF-κB-Inaktivierung benutzen, an der ein Zerfall von IκB-α beteiligt ist. Die unterschiedliche Kinetik des Abbaus läßt vermuten, daß sich die Induktoren hinsichtlich ihres der NF-κB-Aktivierung vorgeschalteten Signaltransduktionsweges unterscheiden. Aufgrund der Tatsache, daß ein polyklonaler Antikörper verwendet wurde, ist es unwahrscheinlich, daß nur ein Epitop von IκB-α nach der Stimulierung verlorenging oder modifiziert wurde. Es ist vielmehr wahrscheinlich, daß der Verlust an IκB-α-Immunreaktivität auf einen raschen und vollständigen Zerfall des Proteins zurückzuführen ist, der zu keinen immunologisch nachweisbaren Abbauprodukten führt. Nach einer Stimulierung mit PMA, IL-1β, LPS oder TNF-α war keine Änderung der elektrophoretischen Beweglichkeit von IκB-α in SDS-Gelen sichtbar, die für einen Hinweis auf eine diesem Zerfall etwa vorausgehende posttranslationale Modifikation von IκB-α geben würde.

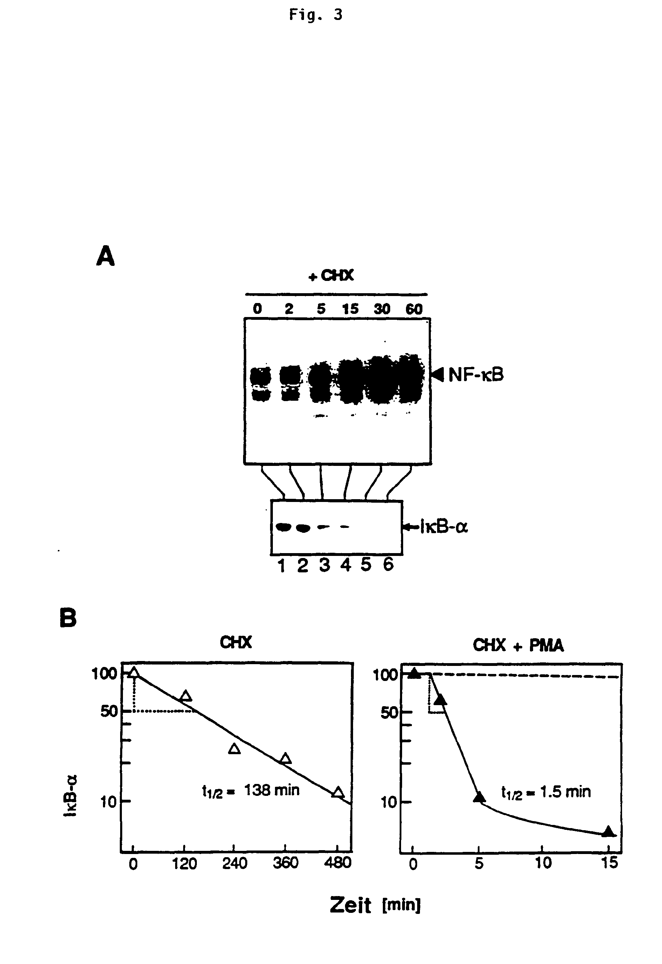

Von den Proteinsyntheseinhibitoren Cycloheximid und Anisomycin ist bekannt, daß auch sie in 70Z/3 Zellen NF-κB aktivieren (Sen und Baltimore, 1986). Die im Rahmen der vorliegenden Erfindung durchgeführte einstündige Behandlung von 70Z/3 Zellen mit Cycloheximid induzierte die Bindung von NF-κB an DNA nur schwach (Fig. 3A, Spur 1, obere Tafel). In diesen Zellen waren mittels Western Blots noch große Mengen IκB-α nachweisbar (Fig. 3A, Spur 1, untere Tafel). Daraus läßt sich ableiten, daß die Inhibierung des normalen IκB-α-Umsatzes für eine effiziente und rasche Aktivierung von NF-κB nicht ausreichend ist. Wenn mit Cycloheximid vorbehandelte Zellen mit PMA induziert wurden, wurde ein rascher Zerfall von IκB-α und daraufhin eine Induktion der Bindung von NF-κB an DNA beobachtet (Fig. 3A); die frühe Kinetik der hier festgestellten IκB-α-Verminderung war von der mit PMA allein beobachteten nicht zu unterscheiden (vgl. Fig. 3A mit Fig. 1B). Der Proteinsyntheseinhibitor Cycloheximid verhinderte jedoch das Wiederauftreten von IκB-α, das 40 min nach der Behandlung mit PMA allein beobachtet worden war (vgl. Fig. 3A mit Fig. 1B), was darauf hindeutet, daß dieses IκB-α neu synthetisiert wurde, vielleicht unter transkriptioneller Kontrolle durch NF-κB selbst.

Ferner wurde die Halbwertszeit von IκB-α in Proteinsynthese-arretierten Zellen ohne oder mit anschließender PMA-Stimulierung der Zellen bestimmt. Die Halbwertszeit von IκB-α. in Cycloheximid-behandelten 70Z/3 Zellen betrug ca. 138 min, wie mittels quantitativer Western Blot-Analyse bestimmt wurde (Fig. 3B). Die Halbwertszeit von IκB-α wurde auf nur 1.5 min im Zeitraum seines raschesten Abbaus während 2 bis 5 min im Anschluß an die PMA-Stimulierung vermindert; dies zeigt, daß PMA einen ca. 90fach verstärkten Abbau von IκB-α induziert.

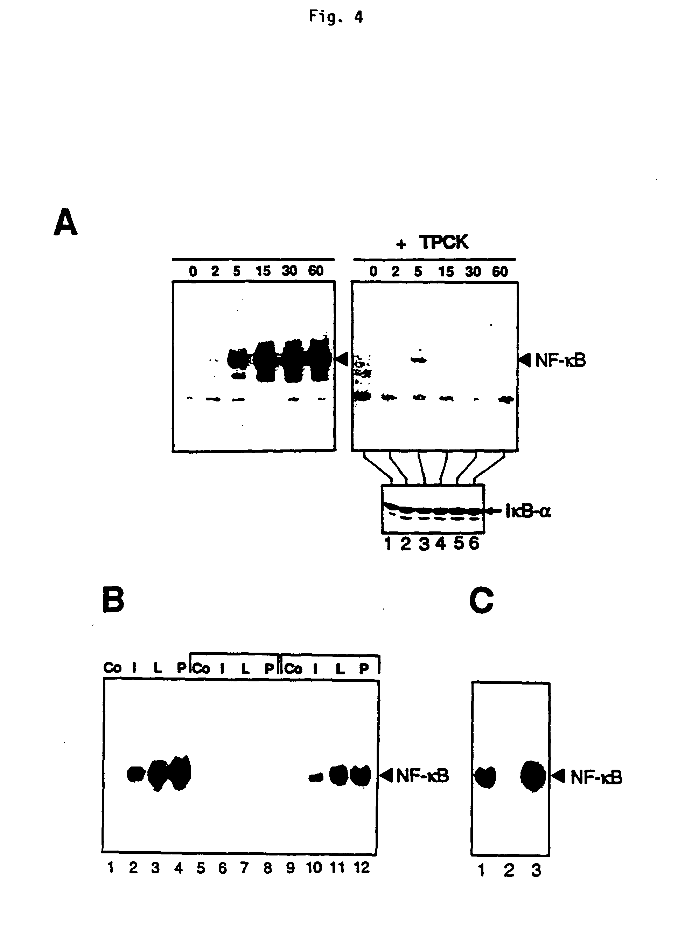

Um festzustellen, ob der induzierbare Zerfall von IκB-α ein für die Aktivierung von NF-κB notwendiger Schritt ist, wurden Zellkulturen der Behandlung mit Proteaseinhibitoren ausgesetzt. Von den verschiedenen Substanzen, von denen gefunden wurde, daß sie aktiv sind, erwies sich der Chymotrypsininhibitor p-Tosyl-L-Phenylalaninchlormethylketon (TPCK) als potentester Inhibitor der NF-κB-Aktivierung in verschiedenen Zelltypen. Eine einstündige Behandlung von 70Z/3 Zellen mit 25 µM TPCK reichte aus, um das Auftreten der NF-κB-DNA-Bindungsaktivität bei einer anschließenden PMA-Behandlung komplett zu unterdrücken (Fig. 4, obere Tafel). Die ID₅₀ von TPCK betrug ca. 8 µM. Die konstitutive DNA-Bindungsaktivität, inklusive Kernfaktor oct-1, wurde nicht signifikant beeinträchtigt (Fig. 4A). Wie im Western Blot gesehen wurde, konnte der Proteaseinhibitor den Zerfall von IκB-α in PMA-behandelten Zellen zur Gänze verhindern (Fig. 4A, untere Tafel). TPCK wirkte sogar hemmend, wenn es zur selben Zeit wie PMA zugesetzt wurde und war gleichermaßen bei der Verhinderung der Aktivierung von NF-κB durch IL-1β und LPS in 70Z/3 Zellen (Fig. 4A, Spuren 6 und 7) und durch TNF-α in verschiedenen anderen Zellinien wirksam. Eine Behandlung von Zellen mit dem Proteaseinhibitor nach Stimulierung mit PMA, IL-1 oder LPS beeinträchtigte die NF-κB-Aktivierung nicht stark, dürfte aber eine weitere Aktivierung arretiert haben (Fig. 4A, Spuren 10-12). Dies zeigt, daß TPCK nicht einfach die DNA-Bindung von NF-κBbeeinträchtigte oder zu einem Zerfall von NF-κB führte, Vorgänge, die bei der Zellyse eingetreten wären. Es ist gleichermaßen unwahrscheinlich, daß TPCK PKC inhibierte, weil von IL-1β und TNF-α gezeigt wurde, daß sie NF-κB unabhängig von PMA-induzierbaren PKC-Isoenzymen aktivieren (Bomsztyk et al., 1991; Meichle et al., 1990).

p-Tosyl-L-Lysinchlormethylketon (TLCK) ist ein Inhibitor von trypsinähnlichen Serinproteasen und in Struktur und chemischer Aktivität sehr mit TPCK verwandt. TLCK konnte in 70Z/3 Zellen die Aktivierung von NF-κB bei einer Konzentration von 25 µM nicht verhindern (Fig. 4B, Spur 3). Es wurde jedoch bei einer Konzentration von 100 µM TLCK eine teilweise Inhibierung der NF-κB-Aktivierung beobachtet. Auch verschiedene andere Proteaseinhibitoren waren wirksam, wenn auch bei höheren Konzentrationen. Der selektive und starke inhibitorische Effekt von TPCK läßt vermuten, daß eine chymotrypsinähnliche Serinprotease bei der Aktivierung von NF-κB beteiligt ist. Diese Protease wird im folgenden als IκB-α-Protease bezeichnet.

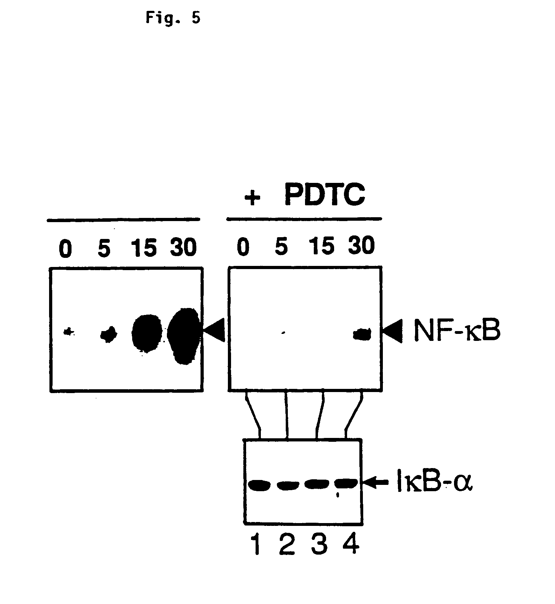

Es wurde kürzlich berichtet, daß reaktive Sauerstoffverbindungen als Botenstoffe bei der Aktivierung von NF-κB durch viele induzierende Faktoren eine Rolle spielen könnten (Schreck et al., 1991; Schreck et al., 1992a,b). Diese Vermutung wurde zum Teil darauf gegründet, daß Antioxidantien wie Thiolverbindungen, Dithiocarbamate und Chelatbildner für freies Eisen die Aktivierung von NF-κB in intakten Zellen unterdrücken. Ein sehr starker Antioxidans-Inhibitor der NF-κB-Aktivierung war Pyrrolidindithiocarbamat (PDTC; Schreck et al., 1992b). Wenn der Zerfall von IκB-α an der Aktivierung von NF-κB maßgeblich beteiligt ist, sollte PDTC ähnlich wie TPCK das Verschwinden von IκB-α verhindern, obwohl diese Inhibierung aufgrund eines Mechanismus ablaufen könnte, der weiter oberhalb der IκB-α-Protease wirkt. 100 µM PDTC unterdrückten wirkungsvoll die Aktivierung der NF-κB-Bindungsaktivität in PMA-behandelten 70Z/3 Zellen (Fig. 5). Wie erwartet, wurde im Western Blot kein signifikanter Abbau des IκB-α Proteins nachgewiesen. Es wurde kürzlich gezeigt, daß PDTC die PMA-induzierte Membranassoziation und Kinaseaktivität von PKC nicht stört, was gegen eine direkte Wirkung von PKC auf IκB-α in intakten Zellen sprach.

Aus den im Rahmen der vorliegenden Erfindung erhaltenen Ergebnissen kann geschlossen werden, daß der proteolytische Abbau von IκB-α als Antwort auf PMA, IL-1B, LPS und TNF-α ein notwendiger Schritt beim Vorgang der NF-κB-Aktivierung ist. Dies ergibt sich nicht nur aus der engen zeitlichen Kopplung des IκB-α-Zerfalls mit der NF-κB-Aktivierung, sondern noch stärker aus der selektiven inhibitorischen Wirkung, die bei niedrigen Konzentrationen des gut charakterisierten Proteaseinhibitors TPCK gesehen wurde. Der induzierbare Zerfall wurde durch eine dramatische Stabilitätsabnahme des Proteins und nicht durch eine Blockierung in seiner neuen Synthese verursacht. Die normale Halbwertszeit der inhibitorischen Untereinheit könnte die schwache Aktivierung von NF-κB durch Proteinsyntheseinhibitoren erklären, aber dies ist eindeutig nicht ausreichend, um eine rasche und vollständige Erschöpfung von IκB-α zu induzieren und NF-κB potent zu aktivieren, wie mit PMA und anderen Stimuli gesehen wurde.

Ein verstärkter Abbau von IκB könnte durch verschiedene Mechanismen kontrolliert werden: Erstens könnte eine neue Modifikation des IκB-Proteins den Inhibitor empfänglicher für einen Abbau durch eine konstitutive Protease gemacht haben. Zweitens könnte es sein, daß eine Protease aktiviert wird, die selektiv IκB abbaut. Ein interessanter Aspekt dieser Möglichkeit ist, daß die IκB-α-Protease oder ein Proteaseinhibitor ein direktes Ziel für Botenstoffe oberhalb davon, wie Proteinkinasen, sein könnte. Drittens könnten sowohl eine Modifikation von IκB als auch eine Induktion einer Proteaseaktivität erforderlich sein. Aufgrund der vorliegenden Ergebnisse kann zwischen diesen Möglichkeiten noch nicht eindeutig unterschieden werden.

Im Rahmen der vorliegenden Erfindung wurde auch die Frage gestellt, in welchem Ausmaß eine direkte Phosphorylierung von IκB an der Freisetzung von IκB und der Aktivierung von NF-κB in intakten Zellen beteiligt ist. Aufgrund der bisher durchgeführten Untersuchungen liegen keine Phosphorylierungsdaten aus intakten Zellen vor, die für eine induzierbare Phosphorylierung von IκB-α sprechen, obwohl eine konstitutive Phosphorylierung von IκB-α beobachtet wurde. Die vorliegenden Ergebnisse können mit den in vitro erhaltenen Kinasedaten (Shirakawa und Mizel, 1989; Ghosh und Baltimore, 1990; Kerr et al., 1991) nur unter der Annahme in Einklang gebracht werden, daß ein Abbau erforderlich ist, um rasch das durch Phosphorylierung freigesetzte IκB zu eliminieren. Ein Abbau könnte erforderlich sein, um IκB, wenn es einmal dephosphoryliert ist, daran zu hindern, von neuem NF-κB zu inhibieren. Angesichts der starken inhibitorischen Wirkung des Proteaseinhibitors TPCK dürfte jedoch eine Phosphorylierung von IκB-α zu transient sein, um NF-κB zu aktivieren und dessen Aufnahme in den Zellkern zu erlauben bzw. um mit den verwendeten Methoden nachgewiesen zu werden.

Ein kontrollierter proteolytischer Abbau von IκB wäre ein ausgezeichneter Mechanismus, die Aktivierung von NF-κB irreversibel zu machen. Die einzige Möglichkeit, aktiviertes NF-κB zu inhibieren, bestünde dann durch neu synthetisiertes IκB. Eine wichtige Frage ist, ob die IκB-α-Protease IκB-α im zytoplasmatischen Komplex mit NF-κB angreift, oder ob vorher eine Freisetzung von IκB von NF-κB erforderlich ist. Es wurde in Versuchen festgestellt, daß überexprimiertes IκB-α nicht signifikant auf eine TNF-α Stimulierung hin abgebaut wird, was hingegen für das endogene zutrifft. Das deutet darauf hin, daß nur das mit NF-κB assoziierte IκB ein Substrat für die IκB-α-Protease ist, wodurch die Notwendigkeit für eine Kennzeichnung, wenn auch nicht für eine Modifikation von IκB-α durch eine direkte Phosphorylierung, obsolet würde. Ein für eine Untereinheit spezifischer Abbau eines Transkriptionsregulators wurde bereits für den α2-Repressor aus Hefe festgestellt (Hochstrasser und Varshavsky, 1990).

Spezifische Inhibitoren der IκB-α-Protease sind als pharmazeutische Wirkstoffe geeignet, um die Aktivierung von NF-κB, die für zahlreiche pathologische Zustände verantwortlich ist, zu verhindern. Beispiele für die zahlreichen biomedizinisch wichtigen Zustände, an denen NF-κB maßgeblich als Signalübermittler und Aktivator von "immediate-early" Genen beteiligt ist, sind die Progression von AIDS, die Aktivierung von T-Zellen, B-Zellen und Makrophagen während der Immunantwort, die sog. "Acute Phase Response", toxischer Schock, Transplantatabstoßung und die Antwort der Zelle auf γ-Bestrahlung und UV-Licht. Spezifische Inhibitoren der IκB-α-Protease sind wirksam als anti-inflammatorische und immunsuppressive Medikamente.

### Figurenübersicht

- Fig. 1:: Schicksal von IκB-α in stimulierten 70Z/3 prä-B-Zellen
- Fig. 2:: Wirkung von IL-1β, LPS und TNF-α auf die Aktivierung von NF-κB und die Stabilität von IκB-α
- Fig. 3:: Wirkung eines Proteinsyntheseinhibitors auf die Stabilität von IκB-α und die Aktivierung von NF-κB in 70Z/3 Zellen
- Fig. 4:: Wirkung des Proteaseinhibitoren TPCK und TLCK auf die Aktivierung von NF-κB und die Stabilität von IκB-α in 70Z/3 Zellen
- Fig. 5:: Wirkung von PDTC auf die Aktivierung von NF-κB und die Stabilität von IκB-α in 70Z/3 Zellen
- Fig. 6:: Wirkung des Proteaseinhibitors TPCK auf die Aktivierung eines Reportergens durch NF-κB in HeLa-Zellen
- Fig. 7:: Wirkung der Proteaseinhibitoren Z-Ile-Glu(Ot-Bu)-Ala-Leucinal und Z-Leu-Leucinal auf die Aktivierung von NF-κB und die Stabilität von IκB-α in HeLa-Zellen
- Fig. 8:: Untersuchung der Proteaseninhibitoren Z-Ile-Glu(Ot-Bu)-Ala-Leucinal und Z-Leu-Leucinal auf hemmende Wirkung des durch TNF-α induzierten proteolytischen Abbaus von IκB-α
- Fig. 9:: Zusammenhang der Induktion der NF-κB-Aktivierung mittels TNF-α und dem Auftreten von phosphoryliertem IκB-α

### Beispiel 1

Schicksal von IκB-α in stimulierten 70Z/3 prä-B-Zellen

Die 70Z/3 Zellen (ATCC No. TIB 158) wurden in RPMI-1640 Medium (Gibco BRL), ergänzt mit 10 % FCS (Gibco BRL) und 50 µM 2-Mercaptoethanol, gezüchtet. 2 ml Aliquots mit ca. 2 - 3 x 10⁶ suspendierten Zellen wurden mit 50 ng/ml PMA (Sigma) verschieden lange behandelt. Die Behandlung wurde durch sofortiges Zentrifugieren (5 sek in einer Eppendorf Microfuge) und Kühlen auf Eis abgebrochen. Die Zellpellets wurden mit 60 µl eines Extraktionspuffers mit hohem Salzgehalt, enthaltend das nichtionische Detergens Nonidet P-40 (Baeuerle und Baltimore, 1988b), lysiert. Die Überstände einer 15 minütigen Zentrifugation bei 13.000 rpm in einer Eppendorf Microfuge wurden mittels Western Blot und EMSA ("electrophoretic mobility shift assay") analysiert. Für SDS-PAGE und Western Blots wurden 30 µl Aliquots der Extrakte mit 15 µl SDS Sample Puffer; Laemmli, 1970) gemischt, gekocht und einer SDS-PAGE auf 12.5 % Polyacrylamid-Minigelen (Biorad) unterworfen. Die Proteine wurden unter Verwendung einer sog. "Semi-Dry"-Blotvorrichtung (Biorad; 1 h bei 15V/2.5mA/cm²) von den Gelen auf Immobilon-P-Filter (0.45 µm; Millipore) übertragen. Die Leistungsfähigkeit des Blots wurde mittels Proteinfärbung der Filter mit Ponceau S (Serva) überprüft. Die Filter wurden über Nacht in Tris-gepufferter Salzlösung, enthaltend 0.1 % (v/v) Tween-20 (TBST), 5 % Magermilchpulver (Nestle) und 1 % BSA blockiert. Daraufhin wurde das Filter eine Stunde lang bei Raumtemperatur mit anti-IκB-α-IgG in einem dialysierten Eluat von der IκB-α-Affinitätssäule, verdünnt 1:100 in Blockierungspuffer, inkubiert. Nach 30 minütigem Waschen in TBST wurde das Filter in einer 1:4000 Verdünnung von Ziegen-anti-Kaninchen IgG/Meerrettichperoxidase-Konjugat (Biorad) in Blockierungspuffer inkubiert. Nach 30 minütigem Waschen in TBST wurde das Filter mit ECL-Nachweisreagens (Amersham) behandelt und exponiert (Kodak XR Film, weniger als 1 min). Für EMSA wurden 2 µl Aliquots der Extrakte zu einer Bindungsmischung hinzugegeben, was eine Endkonzentration von 100 mM KCl, 20 mM HEPES, pH 7.9, 2.5 mM Dithiothreitol, 0.5 mM Phenylmethylsulfonylfluorid (PMSF), 0.2 % Nonidet P-40, 5 % Ficoll, 20 µg BSA, 3 µg Poly(dl-dC) und 10.000 cpm (Cerenkov Zählweise) einer ³²P-markierten doppelsträngigen κB-Oligonukleotid-Sonde (Gibco BRL) ergab. Die Spezifität des Protein-DNA-Komplexes wurde mittels Kompetitionsversuchen und Supershifting mit einem polyklonalen Antikörper, der die C-terminalen 100 Aminosäuren von Rel-A (p65) erkennt, bestätigt. Nach einer 20 minütigen Inkubation auf Eis wurden die Proben einer Elektrophorese auf nativen 4 % Polyacrylamidgelen, gefahren in 0.5 x TBE, unterworfen. Die getrockneten Gele wurden exponiert (Kodak XR Film, -70°C, über Nacht).

Das humane IκB-α wurde in E.coli hergestellt und gereinigt, wie von Zabel et al., 1993, beschrieben. Ein Antiserum gegen IκB-α wurde in Kaninchen hergestellt, das spezifische IgG in 2 ml Serum, affinitätsgereinigt auf IκB-α-Sepharose (Henkel et al., 1992). 2 mg von 6 x His-markiertem, affinitätsgereinigtem humanem IκB-α wurden an 0.5 ml Cyanogenbromid-aktivierte Sepharose 4CL-B (Pharmacia) nach Vorschrift des Herstellers gekoppelt, nach ausgiebigen Waschvorgängen mit PBS und 2 Säulenvolumina 0.1 M Glycin-HCl, pH 2.7, wurden spezifische Antikörper mit 2 Volumina 4 M Guanidinhydrochlorid eluiert. Das Eluat wurde ausgiebig gegen TBST dialysiert.

Das Ergebnis der durchgeführten Versuche ist in Fig. 1 dargestellt. A: Spezifität eines affinitätsgereinigten polyklonalen Kaninchen-anti-IκB-α-Antikörpers in Western Blots. Wie oben angegeben, wurden die Proteine von nichtstimulierten 70Z/3 Zellen in einem Gesamtzellextrakt hoher Salzkonzentration durch Natriumdodecylsulfat-Polyacrylamidgelelektrophorese (SDS-PAGE) aufgetrennt und auf ein Membranfilter übertragen. Ein Streifen des Filters wurde mit anti-IKB-α und einem Peroxidase-markierten anti-Kaninchen-IgG inkubiert (Spur 1). Ein Duplikatfilter wurde mit dem zweiten Antikörper allein inkubiert (Spur 2). Die Figur zeigt die Fluorogramme der Chemilumineszenz-markierten Filter, die Molekulargewichte sind in kDa angegeben. Der Pfeil zeigt die Position einer einzelnen 38 K-Bande an. Als Molekulargewichtsstandards wurden Phosphorylaseb (97), Rinderserumalbumin (BSA, 67), Ovalbumin (45) und Carboanhydrase (30) verwendet. B: Wirkung einer Behandlung von Zellen mit PMA auf IκB-α. Die Zellen wurden für die in der Fig. angegebene Zeitdauer (in min) mit PMA behandelt (Spuren 2-9). Der Nullwert (Spur 1) stammt von unbehandelten Zellen. Nach der Behandlung wurden die Zellextrakte, wie angegeben, einer SDS-PAGE unterworfen und unter Verwendung von anti-IκB-α-IgG mittels Western Blot auf IκB-α untersucht. Der Pfeil zeigt die Position von IκB-α an. Die Figur zeigt einen Ausschnitt eines Flucrogramms.

C: Wirkung einer Behandlung von Zellen mit PMA auf die DNA-Bindungsaktivität von NF-κB. Aliquots von Extrakten aus Kontrollzellen (Spur 1) und PMA-behandelten Zellen (Spuren 2-6; die Behandlungsdauer ist oben in min angegeben; die Ziffern unterhalb der Fig. gibt die Spur an) wurden, wie angegeben, mit der ³²P-markierten DNA-Sonde, die das NF-κB-Bindungsmotiv vom Enhancer des Gens der Maus für die leichte Immunglobulin-k-Kette ("mouse k light chain enhancer"; Sen und Baltimore, 1986) enthielt, inkubiert, worauf eine Analyse der DNA-Bindungsaktivität mittels Gelelektrophorese durchgeführt wurde. Die Fig. zeigt ein Fluorogramm eines nativen Gels. Die gefüllte Pfeilspitze zeigt die Positition des NF-κB-DNA-Komplexes an, die offene Pfeilspitze die Position der nichtkomplexierten DNA-Sonde.

### Beispiel 2

### Wirkung von IL-1β, LPS und TNF-α auf die Aktivierung von NF-κB und die Stabilität von IκB-α

Die in diesem Beispiel verwendeten Methoden, die Kultur der 70Z/3 Zellen sowie die Herstellung von Zellextrakten wurden durchgeführt, wie in Beispiel 1 angegeben. Die 70Z/3 Zellen wurden mit 50 E/ml IL-1β (Boehringer Mannheim) oder mit 15 µg/ml LPS (Sigma) behandelt. Humane HeLa-Zellen wurden in DMEM, ergänzt mit 10 % FCS und 1 % L-Glutamin, gezüchtet und mit 200 E/ml humanem rekombinantem TFN-α (Genzyme) behandelt.

Die Fig. 2 zeigt in der oberen und der mittleren Tafel die Behandlung von 70Z/3 Zellen, behandelt mit IL-1β (Spuren 2-4) und LPS (Spuren 6-8) während der jeweils angegebenen, unterschiedlichen Zeitdauer (in min), gefolgt von einer Untersuchung des Gesamtzellextraktes auf die DNA-Bindungsaktivität von NF-κB und auf die IκB-α-Immunreaktivität unter Verwendung von EMSA bzw. Western Blot. Die untere Tafel zeigt die Ergebnisse der Behandlung von HeLa-Zellen mit TNF-α (Spuren 10-13). Die Fig. zeigt Ausschnitte von Western Blots und Ausschnitte von Fluorogrammen von nativen Gelen. Die Position des NF-κB-DNA-Komplexes in den EMSAs ist durch eine gefüllte Pfeilspitze angegeben. Die Position der IκB-α-Bande auf Western Blots ist durch einen Pfeil angegeben.

### Beispiel 3

Wirkung eines Proteinsyntheseinhibitors auf die Stabilität von IκB-α und die Aktivierung von NF-κB in 70Z/3 Zellen

70Z/3 Zellen wurden mit 25 µg/ml Cycloheximid (Sigma) behandelt (wie von Wall et al., 1986, festgestellt worden war, wird bereits bei einer Konzentration von 10 µg/ml die Proteinsynthese in 70Z/3 Zellen zu 90 % inhibiert). Zellkultur, Extraktherstellung, SDS-PAGE und Western Blot wurden, wie in Beispiel 1 angegeben, durchgeführt. Die densitometrische Untersuchung wurde mit einem Howtek Scanmaster 3 durchgeführt; die Daten wurden mit Hilfe der Software Quantity One Version 2.2 ausgewertet.

Das Ergebnis der durchgeführten Versuche ist in Fig. 3 dargestellt: A: Wirkung von Cycloheximid auf die DNA-Bindung von NF-κB und die zelluläre Konzentration von IκH-α. Die Zellen wurden mit Cycloheximid eine Stunde lang vorbehandelt, worauf eine Stimulierung mit PMA während der angegebenen Zeitdauer (in min) erfolgte. Die obere Tafel zeigt die Untersuchung der Zellextrakte auf die DNA-Bindungsaktivität von NF-κB mittels EMSA, wobei ein Ausschnitt eines Fluorogramms von einem nativen Gel dargestellt ist; die gefüllte Pfeilspitze gibt die Position des NF-κB-DNA-Komplexes an. Die untere Tafel zeigt die Untersuchung von Zellextraktaliquots auf IκB-α mittels Western Blots; die Position der 38 K IκB-α-Bande ist durch einen Pfeil gekennzeichnet. B: Stabilität von IκB-α in Abwesenheit und in Gegenwart von PMA. Die linke Tafel zeigt die Behandlung von Zellen mit Cycloheximid allein; die rechte Tafel zeigt die Behandlung von Zellen mit PMA (Behandlungsdauer in der Fig. angegeben) nach einstündiger Cycloheximid-Vorbehandlung. (Die Gesamtzellextrakte in hoher Salzkonzentration wurden aus Zellkulturaliquots hergestellt, und gleiche Proteinmengen der SDS-PAGE unterworfen. Die IκB-α-Konzentration wurde mittels Western Blot und densitometrischer Quantifizierung der 38 K-Bande in den Fluorogrammen bestimmt.) Die linke Tafel zeigt das nachgewiesene IκB-α (in %) am Beginn der Cycloheximidbehandlung, die rechte am Beginn der PMA-Behandlung.

### Beispiel 4

### Wirkung von Proteaseinhibitoren auf die Aktivierung von NF-κB und die Stabilität von IκB-α in 70Z/3 Zellen

Zellkultur, Extraktherstellung, SDS-PAGE und Western Blot wurden wie in Beispiel 1 durchgeführt. Die Zellen wurden mit 25 µM TPCK oder TLCK (beide von Sigma), gelöst in Dimethylsulfoxid (DMSO) vorbehandelt. Die Kontrollkulturen erhielten eine äquivalente Menge DMSO.

Das Ergebnis der durchgeführten Versuche ist in Fig. 4 dargestellt: A: Wirkung von TPCK auf die Induktion durch PMA. Die Zellen wurden in Abwesenheit (linke Tafel) oder in Gegenwart von TPCK mit PMA nach den angegebenen Zeitspannen behandelt. Die Zellextrakte wurden auf die DNA-Bindungsaktivität von NF-κB mittels EMSA (obere Tafeln) und auf die IκB-α-Proteinmenge mittels Western Blot (untere Tafel) untersucht. Die Position des NF-κB-DNA-Komplexes in einem Ausschnitt eines Fluorogramms von einem nativen Gel ist durch gefüllte Pfeilspitzen gekennzeichnet. Ein Pfeil kennzeichnet die Lage der 38 K IκB-α-Bande im Western Blot. Die schwache untere Bande ist nicht spezifisch; ihre Menge wurde nach Affinitätsreinigung des Antikörpers stark vermindert. B: Wirkung von TPCK auf die Aktivierung von NF-κB durch IL-1β (I), LPS (L) und PMA (P) in 70Z/3 Zellen; in Kontrollzellen (Co). Die Zellen wurden mit TPCK 10 min lang vor der Stimulierung (Spuren 6-8) oder für zusätzliche 10 min nach der Stimulierung (Spuren 10-12; 30 min mit IL-1β und PMA oder 60 min mit LPS) behandelt. Gesamtzellextrakte von Kontrollzellen (Spuren 1-4) und TPCK-behandelten Zellen (Spuren 5-12) wurden mittels EMSA auf die DNA-Bindungsaktivität von NF-κB untersucht; es ist ein Ausschnitt eines Fluorogramms von einem nativen Gel gezeigt. Die gefüllte Pfeilspitze gibt die Position des NF-κB-DNA-Komplexes an. C: Wirkung von TLCK auf die Aktivierung von NF-κB durch PMA. 70Z/3 Zellen wurden unbehandelt gelassen (Spur 1) oder 10 min lang mit entweder 25 µM TPCK (Spur 2) oder 25 µM TLCK (Spur 3) behandelt, worauf der Zusatz von PMA erfolgte. Die Gesamtzellextrakte wurden mittels EMSA auf die DNA-Bindungsaktivität von NF-κB untersucht. Die Fig. zeigt einen Ausschnitt aus dem Fluorogramm; die Position des NF-κB-DNA-Komplexes ist wieder mit einer gefüllten Pfeilspitze gekennzeichnet.

### Beispiel 5

### Wirkung von PDTC auf die Aktivierung von NF-κB und die Stabilität von IκB-α in 70Z/3 Zellen

Zellkultur, Extraktherstellung, EMSA, SDS-PAGE und Western Blot wurden durchgeführt wie in den vorigen Beispielen. Die Behandlung der Zellkulturen mit 100 µM des Ammoniumsalzes von PDTC wurde durchgeführt, wie von Schreck et al. 1992, beschrieben.

Das Ergebnis der durchgeführten Versuche ist in Fig. 5 dargestellt. Die Zellen wurden während der angegebenen Zeiten (in min) mit PMA ohne (linke obere Tafel) oder mit vorheriger einstündiger Inkubation in Gegenwart von PDTC (rechte Tafeln) behandelt. Die Gesamtzellextrakte wurden mittels EMSA auf DNA-Bindungsaktivität von NF-κB (obere Tafel) und mittels Western Blot auf Gehalt an IκB-α-Protein (untere Tafel) untersucht, wobei, wie in den obigen Figuren, Ausschnitte von Fluorogrammen nativer Gele gezeigt und die Lage des NF-κB-DNA-Komplexes durch gefüllte Pfeilspitzen angegeben werden. Ein Ausschnitt eines Western Blots ist dargestellt, in dem die Position der 38 K IκB-α-Bande durch einen Pfeil gekennzeichnet ist.

### Beispiel 6

### Wirkung des Proteaseinhibitors TPCK auf die Aktivierung eines Reportergens durch NF-κB in HeLa-Zellen

Die Zellkultur wurde wie in Beispiel 1 durchgeführt. Die Zellen wurden mit der Calciumphosphat-Methode (Wigler et al., 1978) transfiziert. 10⁵ Zellen pro Schale wurden entweder 1.5 µg Kontrollplasmid, 1.5 µg pUC-TK-Luc oder 1.5 µg pUC-κB-TK-Luc transfiziert. Als Kontrollplasmid wurde das Plasmid pUC19 (ATCC No. 37254, New England Biolabs) verwendet. Das Plasmid pUC-TK-Luc enthält die für Luciferase kodierende Sequenz (De Wet et al., 1987) unter Kontrolle der -105 - +52 Region des Herpes simplex Thymidinkinase-Promotors. Das Plasmid pUC-KB-TK-Luc enthält vor der TK-Promotorregion drei HIV-LTR-Fragmente von je 28 Basenpaaren, enthaltend je zwei KB-Bindungsstellen (Nabel et al., 1988). Die Zellen wurden 20 min vor Stimulation mit TPCK (Sigma) behandelt. Die Lyse der Zellen und der Luciferaseassay wurden nach Brasier et al., 1989, durchgeführt. Die Ergebnisse sind als relative Lichteinheiten (RLE) dargestellt.

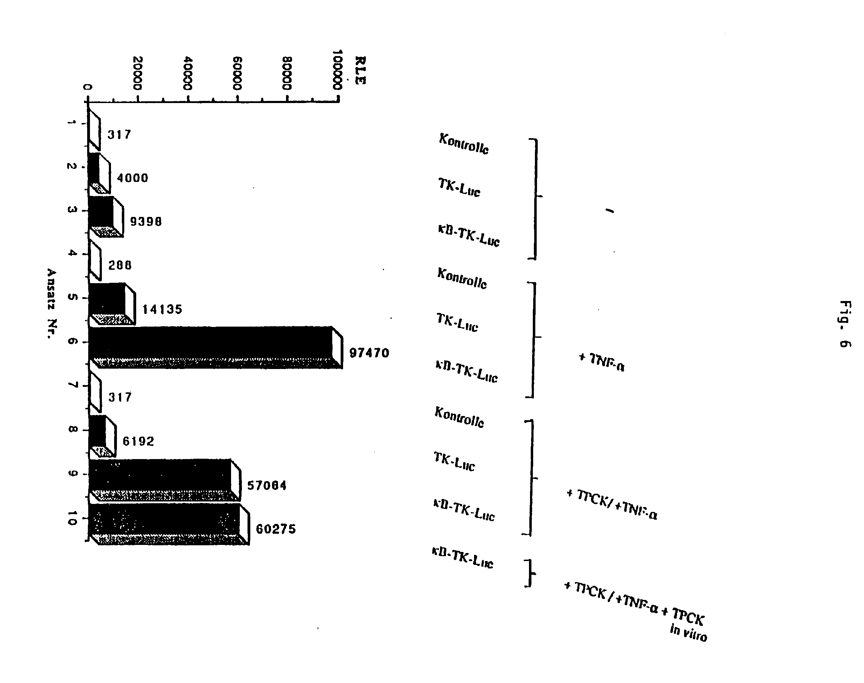

Das Ergebnis der durchgeführten Versuche ist in Fig. 6 dargestellt. Ansätze Nr. 1, 2 und 3 zeigen die Basisexpression von Luciferase in nicht-stimulierten Zellen. Ansätze Nr. 4, 5 und 6 zeigen die Expression von Luciferase nach Stimulation der Zellen mit 200 U/ml menschlichem TNF-α für 3 h. Ansätze Nr. 7, 8 und 9 zeigen die Wirkung von 25 µM TPCK (in DMSO) auf die Luciferaseexpression nach TNF-α Stimulation. In Ansatz Nr. 10 ist die in vitro Wirkung von TPCK auf die Luciferaseaktivität dargestellt. Dazu wurde der Zellextrakt von Ansatz Nr. 9 mit 250 µM TPCK versetzt (vgl. Ansatz Nr. 9 und 10).

### Beispiel 7

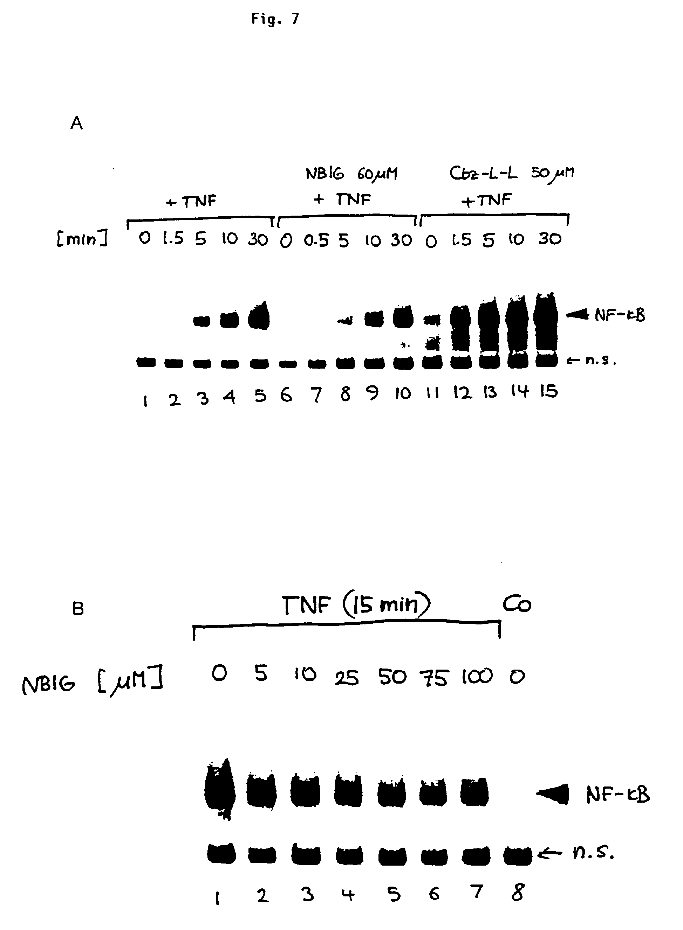

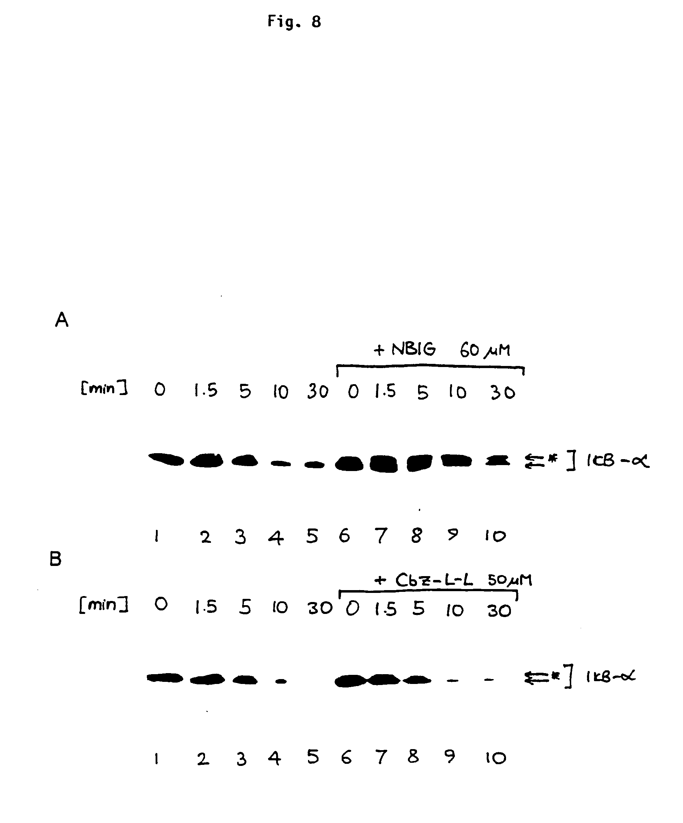

Wirkung der Proteaseinhibitoren Z-Ile-Glu(Ot-Bu)-Ala-Leucinal (NBIG) und Z-Leu-Leucinal auf die Aktivierung von NF-κB und die Stabilität von IκB-α in HeLa-Zellen Die Versuchsanordnung in diesem Beispiel entsprach Beispiel 4; HeLa-Zellen wurden gezüchtet, wie in Beispiel 2 angegeben, als Induktor für die NF-κB-Aktivierung wurde TNF-α verwendet. Als Proteaseinhibitoren wurden Z-Ile-Glu(Ot-Bu)-Ala-Leucinal (NBIG) und die chemisch verwandte Substanz Z-Leu-Leucinal (Cbz-L-L) verwendet (Z steht für Benzyloxycarbonyl).
a) Die elektrophoretischen Mobilitäts-Shiftassays wurden unter Verwendung eines ³²P-markierten Oligonukleotids mit einer NF-κB-Bindungsstelle an Gesamtzellextrakten aus HeLa-Zellen durchgeführt. Fig. 7A zeigt die Verhinderung der Aktivierung von NF-κB nach TNF-α-Stimulierung: der Vergleich der 5- und 10-Minuten Zeitpunkte zeigt deutlich, daß nach Stimulierung mit TNF-α-in Gegenwart von 60 µM NBIG wesentlich weniger NF-κB-DNA-Komplex als in den Kontrollzellen gebildet wird (vgl. die Spuren 3 und 4 mit den Spuren 8 und 9; "n.s." bedeutet in den Fig. 7 und 8 "nicht spezifisch"). Mit Cbz-L-L trat dieser Effekt nicht auf (Spuren 11 bis 15). Der ID₅₀-Wert für NBIG lag bei ca. 50 µM (Fig. 7B).
b) Die Western Blot-Analyse zeigte, daß TNF-α den proteolytischen Abbau von IκB-α induziert (Fig. 8, Spuren 3 bis 5). In Gegenwart von 60 µM NBIG ist dieser Abbau stark vermindert (Fig. 8, vgl. Spuren 4 und 9, 10-Minutenwert). Die Hemmung der NF-κB-Aktivierung nach Stimulation mit TNF-α geht demnach mit einer Hemmung des Abbaus von IκB-α einher (Fig. 8A). Im Gegensatz zum Proteaseinhibitor TPCK (vgl. Beispiel 4) wies NBIG die Besonderheit auf, bei einer Konzentration von 60 µM eine Akkumulation einer modifizierten, im SDS-Gel langsamer laufende Form von IκB-α herbeizuführen (Fig. 8A, vgl. die Spuren 4 und 9. Der Inhibitor Cbz-L-L (50 µM) zeigte diese Effekte nicht (Fig. 8B). In der Figur ist die langsamer wandernde Form von IκB-α mit einem Stern gekennzeichnet.

Die Inkubation von Zellfraktionen mit saurer Phosphatase aus Kartoffeln bewirkte, daß die langsamer migrierende Form von IκB-α wieder in die schneller migrierende überführt wurde. Dieser Befund zeigt, daß es sich bei der langsamer wandernden Form in IκB-α, die nach Stimulierung durch TNF-α unter Einwirkung von NBIG akkumuliert wird, um eine phosphorylierte Form in IκB-α handelt.

In einem weiteren Experiment wurde NBIG zusammen mit dem Phosphatasehemmer Okadasäure (100 nM) angewendet. Die Anwesenheit von Okadasäure bewirkte ein vollständiges Veschwinden der schneller wandernden, nicht-phsophorylierten Form von IκB-α.

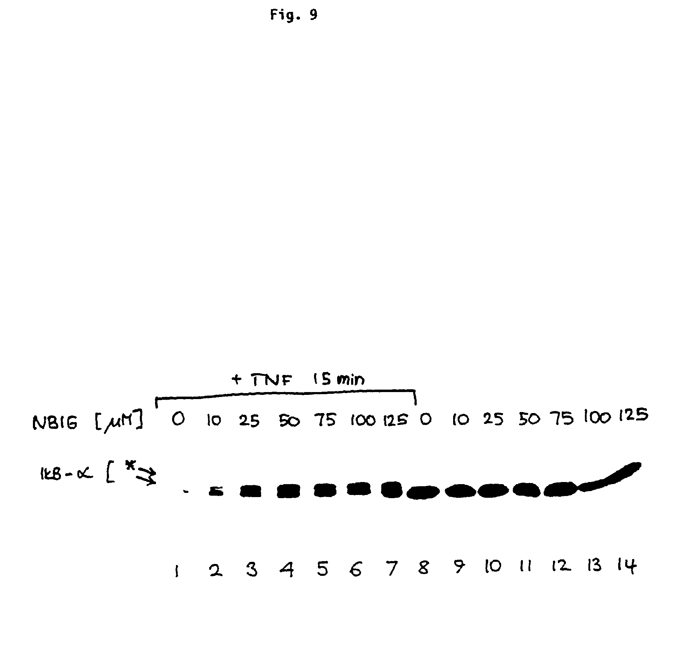

Die Phosphorylierung von IκB-α wird nicht durch die Behandlung mit NBIG induziert (Fig. 9, Spuren 8 bis 14), nicht einmal bei einer Konzentration von 125 µM (Spur 14). Die phosphorylierte Form von IκB-α entsteht somit nur, wenn die Zellen mit TNF-α induziert werden (Fig. 9, Spuren 2 bis 7), wobei die Menge an modifiziertem IκB-α mit der Konzentration von NBIG zunimmt. Die Beobachtung, daß das Auftreten und die Stabilisierung der phosphorylierten Form in IκB-α nicht zeitlich mit der Aktivierung von NF-KB korreliert (vgl. Fig. 8A, Spuren 6 bis 9, mit Fig. 7A, Spuren 6 bis 9), bestärkt die Annahme, daß eine Phosphorylierung von IκB-α in intakten Zellen nicht für eine Aktivierung von NF-κB ausreicht.

### Beispiel 8

Inhibierung der Expression der NF-κB-regulierten Zytokine IL-6 und IL-8 in HeLa-Zellen durch den Proteaseinhibitor NBIG

Das Prinzip der Versuchsanordnung entsprach den vorangegangenen Beispielen.

Die Zellen wurden - mit oder ohne 30 min Vorbehandlung mit 100 µmol NBIG - mit TNF (200 E/ml) oder mit PMA (50 ng/ml) stimuliert und die Konzentration von IL-6 bzw. IL-8 in den Zellkulturüberständen mittels ELISA (British Biotechnology Products Ltd.) zu verschiedenen Zeitpunkten gemessen. Die Expressionswerte sind in der Tabelle wiedergegeben. Die erhaltenen Werte zeigen, daß nach Stimulierung von 120 min in Gegenwart des Proteaseinhibitors die Produktion von IL-6 um 79 % bzw. 73 % (TNF-Stimulierung) und die Produktion von IL-8 um 90 % bzw. 98 % (PMA-Stimulierung) inhibiert wird.

**Tabelle**

| | IL-6 | (pg/ml) | | IL-8 | (pg/ml) | |
|---|---|---|---|---|---|---|
| | 0' | 15' | 120' | 0' | 15' | 120' |
| TNF | - | 875 | 6750 | - | - | 52 |
| TNF+NBIG | - | 875 | 1438 | - | - | 5 |
| PMA | - | 750 | 12813 | - | - | 410 |
| PMA+NBIG | - | 1125 | 3438 | - | - | 10 |

### Literatur

Baeuerle, P.A. und Baltimore, D., 1988a, Cell 53, 211-217.

Baeuerle, P.A. und Baltimore, D., 1988b, Science 242, 540-546.

Baeuerle, P.A., 1991, Biochim. Biophys. Acta 1072, 63-80.

Baeuerle, P.A. und Baltimore, D., 1991, In: Molecular Aspects of Cellular Regulation Vol. 6, Hormonal Control Regulation of Gene Transcription, Cohen, P. und Foulkes, J.G. (eds.), Elsevier/North Holland Biomedical Press, Amsterdam, pp. 409-432.

Blank, V., Kourilsky, P. und Israel, A., 1992, Trends Biochem. Sci. 17, 135-140.

Bomsztyk, K. et al., 1991, Cell. Regul. 2, 329-337.

Brasier, A.R., Tate, J.E. und Habener, J.F., 1989, BioTechniques 7, 1116-1122.

De Wet, J.R., Wood, K., DeLuca, M., Helsinki, D. und Subramani, S., 1987, Mol. Cell. Biol. 7, 725-737.

Gosh, S. und Baltimore, D., 1990, Nature 344, 678-682.

Grimm, S. und Baeuerle, P.A., 1993, Biochem. J. 290, 297-308.

Gritz, L. und Davies, J., 1983, Gene 25, 179-188.

Hartmann, A., 1991, BioTec 5, 40-45.

Haskill et al., 1991, Cell 65, 1281-1289.

Henkel et al., 1992, Cell 68, 1121-1133.

Hochstrasser, M. und Varshavsky, A., 1990, Cell 61, 697-708.

Kerr et al., 1991, Genes Dev. 5, 1464-1476.

Kricka, L.J., 1988, Analyt. Biochem. 175, 14-21.

Laemmli, U.K., 1970, Nature 227, 680-685.

Meichle, A., Schütze, S., Hensel, G., Brunsing, D. und Krönke, M., 1990, J. Biol. Chem. 265, 8339-8347.

Mulligan, R. und Berg, P., 1981, Proc.Natl.Acad.Sci. USA 78, 2072-2076.

Nabel et al., 1988, Science 239, 1299-1301.

Nolan, G.P. und Baltimore, D., 1992, Curr. Opin. Genet. Dev. 2, 211-220.

Orlowski, M., 1990, Biochemistry 29, 10289-10297.

Remington's Pharmaceutical Sciences, 1980, Mack Publ.

Co., Easton, PA, Osol (ed.). Rivett, A.J., 1989, Arch. Biochem. Biophys. 268, 1-8. Schreck, R., Rieber, P. und Baeuerle, P.A., 1991,

EMBO J. 10, 2247-2258.

Schreck, R., Albermann, K. und Baeuerle, P.A., 1992a,

Free Rad. Res. Comms. 17, 221-237.

Schreck, R., Meier, B., Männel, D., Droge, W. und

Baeuerle, P.A., 1992b, J. Exp. Med. 175, 1181-1194. Shirakawa, F. und Mizel, S., 1989, Mol. Cell. Biol. 9, 2424-2430.

Sen, R. und Baltimore, D., 1986, Cell 47, 921-928.

Southern, P. und Berg, P., 1982, J. Mol. Appl. Gen. 1, 327.

Subramani, S. und DeLuca, M., 1987, Genetic

Engineering, Principles and Methods, J.K. Sedlow ed., Plenum Press, New York, Band 10, 75-89.

Tanahashi, H., Ito, T., Inouye, S., Tsuji, F.I. und Sakaki, Y., 1990, Gene 96, 249-255.

Wall, R. et al., 1986, Proc.Natl.Acad.Sci. USA 83, 295-298.

Wieland, E. et al., 1985, Ärztl. Lab. 31, 203-214.

Wigler et al., 1978, Cell 14, 725-731.

Zabel, U., Henkel, T., dos Santos Silva, M. und Baeuerle, P.A., 1993, EMBO J. 12, 201-211.

## Patentansprüche

1. Verfahren zum Identifizieren von Substanzen, die die NF-κB-Aktivierung inhibieren, **dadurch gekennzeichnet, daß** man die Testsubstanzen auf ihre Fähigkeit untersucht, den proteolytischen Abbau von IκB-α spezifisch zu inhibieren, indem man ein IκB-α enthaltendes Substrat in Gegenwart einer Testsubstanz mit einer Zubereitung behandelt, die proteolytische Aktivität für IκB-α aufweist, und bestimmt, ob und in welchem Ausmaß die Testsubstanz den proteolytischen Abbau von IκB-α inhibiert.

2. Verfahren nach Anspruch 1, **dadurch gekennzeichnet, dass** man im Anschluß bzw. parallel in Gegenwart der Testsubstanz die NF-κB-Aktivierung in höheren eukaryotischen, insbesondere humanen, mit einem auf NF-κB-Aktivierung ansprechenden Reportergenkonstrukt transformierten Zellen induziert und die Expression des Reportergens mißt.

3. Verfahren nach Anspruch 1, **dadurch gekennzeichnet, daß** man ein zellfreies System einsetzt.

4. Verfahren nach Anspruch 3, **dadurch gekennzeichnet, daß** man als IκB-α enthaltendes Substrat rekombinantes IκB-α, gebunden an einen festen Träger, einsetzt.

5. Verfahren nach Anspruch 4, **dadurch gekennzeichnet, daß** man als IκB-α enthaltendes Substrat rekombinantes IκB-α in Lösung einsetzt.

6. Verfahren nach Anspruch 4, **dadurch gekennzeichnet, daß** man als IκB-α enthaltendes Substrat phosphoryliertes IκB-α einsetzt.

7. Verfahren nach Anspruch 6, **dadurch gekennzeichnet, daß** man phosphoryliertes IκB-α einsetzt, das erhalten wurde durch Behandlung von Zellen mit einer die NF-κB-Aktivierung induzierenden Substanz und mit einer Substanz, die den protealytischen Abbau von IκB-α hemmt und die Akkumulierung der phosphorylierten Form von IκB-α bewirkt.

8. Verfahren nach Anspruch 1, **dadurch gekennzeichnet, daß** man als Zubereitung, die proteolytische Aktivität für IκB-α aufweist, einen Extrakt aus Zellen einsetzt, die mit einem Induktor für die NF-κB-Aktivierung behandelt wurden, oder eine Fraktion eines solchen Extraktes.

9. Verfahren nach Anspruch 2 und 4, **dadurch gekennzeichnet, daß** man in einem zellfreien System rekombinantes IκBα als Substrat einsetzt und humane, mit einem auf NF-κB-Aktivierung ansprechenden Reportergenkonetrukt transformierte, mit einem Induktor für die NF-κB-Aktivierung stimulierte Zellen mit der Testsubstanz behandelt und die Expression des Reportergens mißt.

10. Verfahren nach Anspruch 9, **dadurch gekennzeichnet**, daas das Reportergen ein Luciferasegen ist.

11. Verfahren nach Anspruch 9 oder 10, **dadurch gekennzeichnet, daß** das Reportergenkonstrukt mindestens zwei κB-Motive enthält.

## Claims

1. Method of identifying substances which inhibit NF-κB activation, **characterised in that** the test substances are investigated for their ability to specifically inhibit the proteolytic breakdown of IκB-α, by treating a substrate containing IκB-α in the presence of a test substance with a preparation which has proteolytic activity for IκB-α, and determining whether and to what extent the test substance inhibits the proteolytic breakdown of IκB-α.

2. Method according to claim 1, **characterised in that** subsequently or in parallel, in the presence of the test substance, the NF-κB activation is induced in higher eukaryotic, particularly human, cells transformed with a reporter gene construct that responds to NF-κB activation and the expression of the reporter gene is measured.

3. Method according to claim 1, **characterised in that** a cell-free system is used.

4. Method according to claim 3, **characterised in that** recombinant I6B-α bound to a solid carrier is used as the substrate containing IκB-α.

5. Method according to claim 4, **characterised in that** recombinant IκB-α in solution is used as the substrate containing IκB-α.

6. Method according to claim 4, **characterised in that** phosphorylated IκB-α is used as the substrate containing IκB-α.

7. Method according to claim 6, **characterised in that** phosphorylated IκB-α is used which has been obtained by treating cells with a substance which induces NF-κB activation and with a substance which inhibits the proteolytic breakdown of IκB-α and brings about the accumulation of the phosphorylated form of IκB-α.

8. Method according to claim 1, **characterised in that** an extract from cells which have been treated with an inducer for NF-κB activation, or a fraction of such an extract, is used as the preparation which has proteolytic activity for IκB-α.

9. Method according to claims 2 and 4, **characterised in that** in a cell-free system recombinant IκB-α is used as substrate and human cells transformed with a reporter gene construct that responds to NF-κB activation and stimulated with an inducer for NF-κB activation are treated with the test substance and the expression of the reporter gene is measured.

10. Method according to claim 9, **characterised in that** the reporter gene is a luciferase gene.

11. Method according to claim 9 or 10, **characterised in that** the reporter gene construct contains at least two κB motifs.

## Revendications

1. Procédé pour identifier des substances qui inhibent l'activation de NF-κB **caractérisé en ce que** l'on examine les substances test concernant leur capacité à inhiber spécifiquement la dégradation protéolytique de IκB-α en traitant un substrat contenant IκB-α en présence d'une substance test avec une préparation qui présente une activité protéolytique pour IκB-α et en déterminant si et dans quelle mesure la substance test inhibe la dégradation protéolytique de IκB-α.

2. Procédé selon la revendication 1 **caractérisé en ce que**, ensuite ou parallèlement, on induit en présence de la substance test l'activation de NF-κB dans des cellules eucaryotes supérieures, en particulier humaines, transformées avec une construction de gène rapporteur réagissant à l'activation de NF-κB et on mesure l'expression du gène rapporteur.

3. Procédé selon la revendication 1 **caractérisé en ce que** l'on utilise un système sans cellules.

4. Procédé selon la revendication 3 **caractérisé en ce que** l'on utilise comme substrat contenant IκB-α IκB-α recombinant lié à un support solide.

5. Procédé selon la revendication 4 **caractérisé en ce que** l'on utilise comme substrat contenant IκB-α IκB-α recombinant en solution.

6. Procédé selon la revendication 4 **caractérisé en ce que** l'on utilise comme substrat contenant IκB-α IκB-α phosphorylé.

7. Procédé selon la revendication 6 **caractérisé en ce que** l'on utilise IκB-α phosphorylé qui a été obtenu par traitement de cellules avec une substance induisant l'activation de NF-κB et avec une substance qui inhibe la dégradation protéolytique de IκB-α et qui provoque l'accumulation de la forme phosphorylée de IκB-α.

8. Procédé selon la revendication 1 **caractérisé en ce que** l'on utilise comme préparation qui présente une activité protéolytique pour IκB-α un extrait de cellules qui ont été traitées avec un inducteur pour l'activation de NF-κB ou une fraction d'un tel extrait.

9. Procédé selon les revendications 2 et 4 **caractérisé en ce que** l'on utilise IκB-α recombinant comme substrat dans un système sans cellules et on traite avec la substance test des cellules humaines transformées avec une construction de gène rapporteur réagissant à l'activation de NF-κB, stimulées avec un inducteur pour l'activation de NF-κB et on mesure l'expression du gène rapporteur.

10. Procédé selon la revendication 9 **caractérisé en ce que** le gène rapporteur est un gène de luciférase.

11. Procédé selon la revendication 9 ou 10 **caractérisé en ce que** la construction de gène rapporteur contient au moins deux motifs κB.
